(19) Europäisches Patentamt
European Patent Office
Office européen des brevets

(11) Publication number: **0 097 460 B1**

(12) **EUROPEAN PATENT SPECIFICATION**

(45) Date of publication of patent specification: **06.04.88**

(21) Application number: **83303353.3**

(22) Date of filing: **09.06.83**

(51) Int. Cl.⁴: **C 07 D 213/64,**
C 07 D 213/70, A 01 N 43/40
// C07D213/84, C07D213/79,
C07D213/61

(54) **Pyridyl(oxy/thio)phenoxy compounds, herbicidal compositions and methods.**

(30) Priority: **18.06.82 US 389840**

(43) Date of publication of application:
**04.01.84 Bulletin 84/01**

(45) Publication of the grant of the patent:
**06.04.88 Bulletin 88/14**

(84) Designated Contracting States:
**AT BE CH DE FR IT LI NL SE**

(56) References cited:
**EP-A-0 000 483**
**EP-A-0 001 473**
**EP-A-0 003 313**
**EP-A-0 003 877**
**EP-A-0 021 453**
**EP-A-0 050 097**
**FR-A-2 412 532**
**FR-A-2 419 285**

**The file contains technical information submitted after the application was filed and not included in this specification**

(73) Proprietor: **THE DOW CHEMICAL COMPANY**
**2030 Dow Center Abbott Road P.O. Box 1967**
**Midland, MI 48640 (US)**

(72) Inventor: **Johnston, Howard**
**430, Las Lomas Way**
**Walnut Creek California (US)**
Inventor: **Troxell, Lillian Heitz**
**1715 Hillcrest**
**Antioch California (US)**

(74) Representative: **Allard, Susan Joyce et al**
**BOULT, WADE & TENNANT 27 Furnival Street**
**London EC4A 1PQ (GB)**

**EP 0 097 460 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates (a) to novel pyridyl-(oxy/thio)phenoxy compounds, (b) to herbicidal compositions of such novel compounds and (c) to methods of using such compounds for the preemergent and postemergent control of grassy weeds in non-crop areas as well as in the presence of certain valuable crops such as soybeans, cotton and wheat.

Belgian Patent No. 834,495, issued February 2, 1976, as well as the published German patent application equivalent thereto, viz., No. 2,546,251, published April 29, 1976, describe 2-(4-(pyridinyl-2-oxy)phenoxy)alkanoic acids, salts and esters having halo substitution in the 3- and/or 5-ring positions in the pyridine ring. Later references, e.g. published British Patent Application 2,026,865 disclose such compounds having trifluoromethyl substitution on the pyridine ring and European Patent Application 0002800 describes the enhanced effect of the D-stereoisomers of such compounds.

FR—A—2419285 describes certain 2-(4-pyridinyl-2-oxy)phenoxy) valeric acids and their derivatives which have a trifluoromethyl substituent at position 5 of the pyridine ring and optionally have a halogen substituent at position 3 of the pyridine ring.

EP—A—0050097 describes certain 2-(4-(pyridyl-2-oxy)phenoxy)propanoic acids and their derivatives which have a trifluoromethyl substituent at position 5 of the pyridine ring and optionally a halogen substituent at position 3 of the pyridine ring.

The present invention is directed to novel pyridyl(oxy/thio)phenoxy compounds having herbicidal activity and which correspond to the formula:

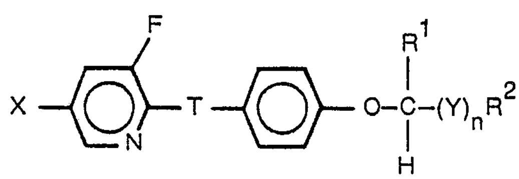

wherein

X is $CF_3$, $CHF_2$ or $CClF_2$:

T is O or S;

Y is an alkyl, alkenyl or alkynyl group containing an even number of from 2 to 18 carbon atoms;

n is 0 or 1;

$R^1$ is a $C_1$—$C_3$ alkyl group; and

$R^2$ is a carboxyl group; an alkali, alkaline earth metal, ammonium or organic amine salt thereof; or an organic group that can be hydrolyzed and/or oxidized in plants or the soil to a carboxyl group that is in undissociated and/or dissociated form.

The invention is also directed to the novel stereoisomers of such compounds, the *R*-isomers having exceptional activity.

In a preferred aspect the present invention provides a herbicidally active compound or a resolved optically active isomer thereof having the formula:

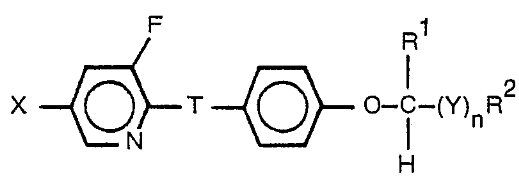

wherein

X is $CF_3$, $CHF_2$ or $CClF_2$:

T is O or S;

Y is an alkyl, alkenyl or alkynyl group containing an even number of from 2 to 18 carbon atoms;

n is 0 or 1;

$R_1$ is a $C_1$—$C_3$ alkyl group; and

$R_2$ is a carboxylic acid group; an alkali, alkaline earth metal, or ammonium or organic amine salt thereof; or a $C_1$—$C_6$ lower alkyl ester thereof.

In a still further preferred aspect the present invention provides a herbicidally active compound or a resolved optically active isomer thereof having the formula:

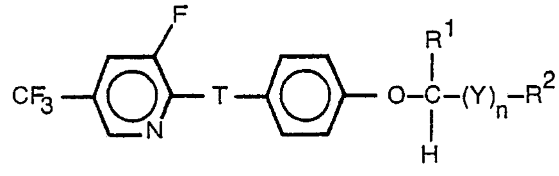

wherein

T is O or S;

Y is an alkyl, alkenyl or alkynyl group containing an even number of from 2 to 18 carbon atoms;

n is 0 or 1;

$R^1$ is a $C_1$—$C_3$ alkyl group; and

$R^2$ is a carboxylic acid group;

an alkali or alkaline earth metal salt thereof; or a $C_1$—$C_6$ lower alkyl ester or $C_1$—$C_6$ thiolester thereof; or the group

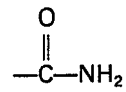

—CN or —$CH_2OH$.

A variety of herbicidal compounds containing substituted pyridyl and phenoxy moieties joined via a bivalent —O— and —S— are described in the art. For examplle, U.S. Patent Nos. 4,046,553; 4,317,913; 4,267,336; 4,213,774; 4,324,627 and 4,309,547 and European Patent 483 all describe such compounds, methods of making them, compositions containing them and methods of utilizing said compositions. In general, the moieties bonded to the pendant —O— group of the phenoxy in the herbicidal compounds described in these references will also be suitable as the monovalent organic radical represented by the group

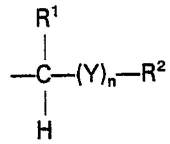

in the formula for the aforementioned novel compounds and, given the appropriate pyridine starting material, the compounds of this invention can be prepared by methods described in the above-mentioned prior art, and can be utilized in compositions as described in the prior art.

By the term "undissociated and/or dissociated form" as used herein is meant that the carboxyl group may be present in ionized form as well as unionized form in the plants or the soil.

In the group

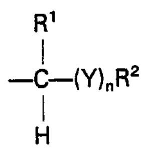

Y is an alkyl, alkenyl or alkynyl group containing an even number of from 2 to 18 carbon atoms, n is 0 or 1, $R^1$ is a $C_1$—$C_3$ alkyl group and $R^2$ is preferably a group corresponding to one of the following formulae:

—CN, 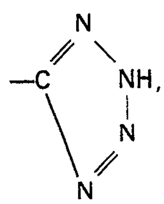 , 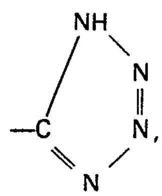 , 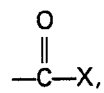 ,

wherein X is halogen, or CN,

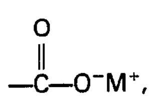 ,

wherein M is a metallic cation, ammonium or an organic amine cation typically, but not exclusively, containing alkyl (saturated or unsaturated), alicyclic, heterocyclic or aromatic groups, all unsubstituted or substituted with various other groups not limited to, but including, halo, cyano, nitro and unsubstituted or substituted thiol, hydroxy, amino or carboxyl groups and, additionally, alicyclic, heterocyclic and aromatic groups substituted with unsubstituted or substituted saturated or unsaturated alkyl groups, for example, trifluoromethyl, chloromethyl, cyanomethyl and vinyl,

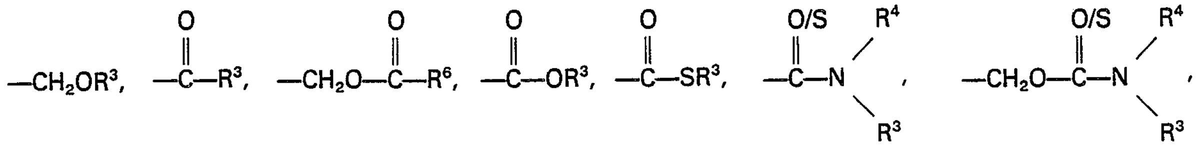

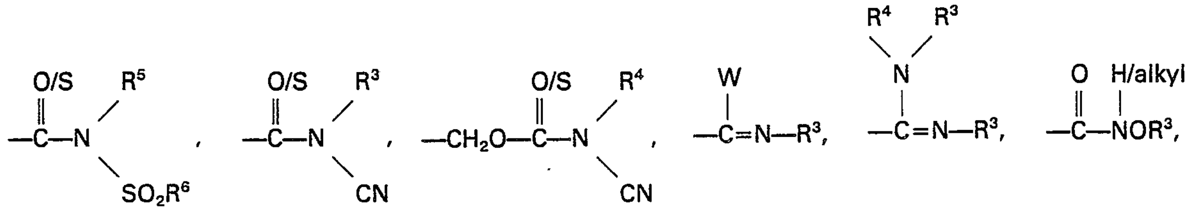

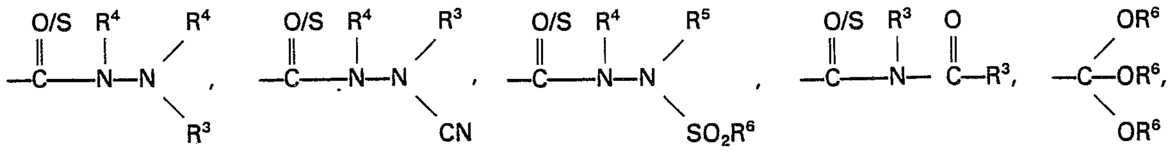

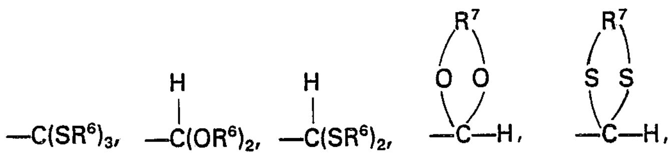

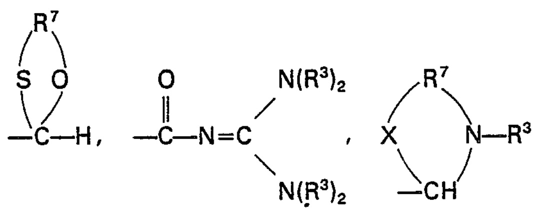

where X is S or O,
where W is halogen; $R^3$ is H or $R^6$; $R^4$ is H, alkoxy or $R^6$; $R^5$ is H, a metallic cation or $R^6$; and $R^6$ is an alkyl (saturated or unsaturated); alicyclic, heterocyclic or aromatic group, unsubstituted or substituted with various other groups not limited to, but including, halo, cyano, nitro and unsubstituted or substituted thiol, hydroxy, amino or carboxyl groups and, additionally, alicyclic, heterocyclic and aromatic groups substituted with unsubstituted or substituted saturated or unsaturated alkyl groups, for example, trifluoromethyl, chloromethyl, cyanomethyl and vinyl,

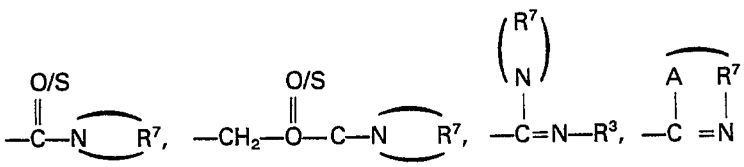

where A is O, S or N, or

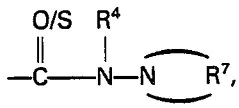

where $R^7$ completes an unsubstituted or substituted saturated heterocyclic ring system.

The above derivatives can be made by processes generally known to those skilled in the art and as described in the above-mentioned patents. For example, the corresponding acid chlorides can be reacted with a Grignard reagent to make the desired aldehyde or ketone derivative. Similarly, reaction of an acid chloride with KSH will provide the desired thiol acid. Thioamides may be prepared from the corresponding amide by reaction with $P_2S_5$ or, if hydrogen is present on the nitrogen atom, the carbonyl may be converted to, e.g., chloride, with removal of HCl, followed by reaction with hydrogen sulfide. Carbamoyl chlorides are

available in the art or they may be prepared from the desired amine and phosgene or thiophosgene for use in making compounds containing the

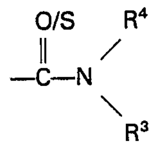

group.

The reaction of an amine with a sulfonyl chloride, e.g., $R^5NH_2 + R^6SO_2Cl$ provides the group

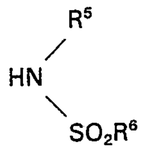

for use in reacting with an appropriate acid chloride.

The reaction of an amine with BrCN provides, e.g.,

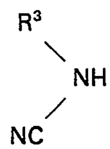

which reacts with the appropriate acid chloride to provide compounds containing the

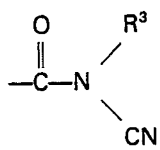

moiety. $P_2S_5$ is employed to make the corresponding S-containing compound.

Reaction of the above cyanoamine with phosgene or thiophosgene provides

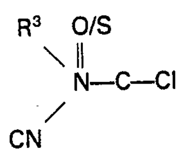

for use in making the corresponding derivatives.

The reaction of the compounds having the moiety

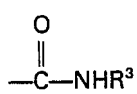

with $PCl_5$ will provide compounds having the moiety

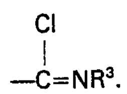

The reaction of the corresponding acid chloride with $RONH_2$ will provide compounds having the group

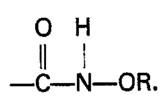

Various hydrazine derivatives can be made, e.g., from trimethyl hydrazine by reaction with the acid chlorides. The reaction of the amides, e.g.,

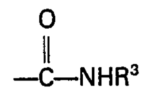

with dicarboxylic anhydrides will provide compounds having the group

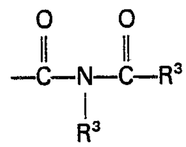

$R^2$ is preferably a carboxylic acid group, an alkali or alkaline earth metal salt thereof, an ammonium or organic amine salt thereof or a lower alkyl ester thereof. By the term "lower alkyl" is meant straight, branched or cyclic saturated alkyl groups containing up to 6 carbon atoms. Preferably, n is 0.

In the above formulae the aliphatic groups preferably contain 1 to 6 carbon atoms, the alkenyl and alkynyl groups preferably contain 2 to 6 carbon atoms, the alicyclic groups preferably contain 3 to 6 carbon atoms and the aromatic moiety is preferably phenyl, although other ring systems, including heterocyclic ring systems, may be employed if desired.

In the formula for the aforementioned novel compounds, T is preferably O and X is preferably $CF_3$. Most preferred are the compounds in which X is $CF_3$, T is 0, and the group

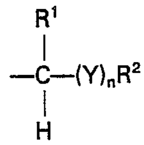

is a 2-propanoic acid methyl, ethyl, propyl, isopropyl, isobutyl or n-butyl ester.

The R-enatiomers of the compounds of the invention are particularly preferred.

The compounds of the above formula, hereinafter referred to for convenience as "active ingredients", have been found to be especially active as herbicides for the control of undesired vegetation, for example, grassy or graminaceous weeds and are unexpectedly more effective than the compounds of the known art. Especially surprising is the finding that the unexpected activity of the presently claimed compounds is specifically related to fluorine substituted in the 3-position of the pyridine ring; fluorine substitution in other positions on the pyridine ring, e.g. the 5-position, does not cause unusually beneficial activity to result. With the compounds of this invention, it is possible to employ lower dosage rates and still obtain effective control, thus reducing plant residues and any potential environmental contamination and/or toxicological effect on fish and warm blooded animals. Accordingly, the present invention also encompasses herbicidal compositions containing one or more of these active ingredients as well as preemergent and postemergent methods of controlling undesired plant growth, especially in the presence of valuable crops. Such methods comprise applying a herbicidally-effective amount of one or more of said active ingredients to the locus of the undesired plants, that is, the seeds, foliage, rhizomes, stems and roots or other parts of the growing plants or soil in which the plants are growing or may be found. The compositions are generally applied at a dose rate sufficient to provide from 0.011 to 22.4 Kgs/hectare (0.01 to 20lbs per acre) of the active-ingredient.

The term "herbicide" is used herein to mean an active ingredient which controls or adversely modifies the growth of plants because of phytotoxic or other effects substantial enough to seriously retard the growth of the plant or further to damage the plant sufficiently to kill the plant.

By "growth controlling" or "herbicidally-effective" amount is meant an amount of active ingredient which causes a modifying effect and includes deviations from natural development, killing, regulation, desiccation, retardation, and the like.

The term "plants" is meant to include germinant seeds, emerging seedlings, rhizomes, stolons and other underground propagules, and established vegetation.

The active ingredients, i.e., new compounds, of the present invention are readily prepared by processes described in the above prior art by choosing the appropriate starting materials.

The present invention thus includes within its scope a process for the preparation of a compound of the formula:

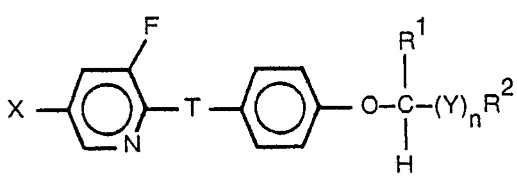

wherein X, T, Y, n, $R^1$ and $R^2$ are as hereinbefore defined which process comprises reacting a compound of the formula:

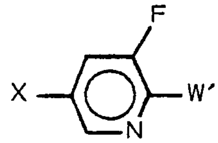

wherein W' is F, Cl or Br and X are as hereinbefore defined with a compound having the formula:

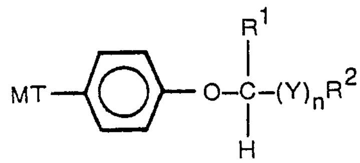

wherein M is hydrogen or a metallic cation, and T, Y, n, $R^1$ and $R^2$ are as hereinbefore defined in the presence of a base.

The present invention also includes within its scope another process for the preparation of a compound of the formula:

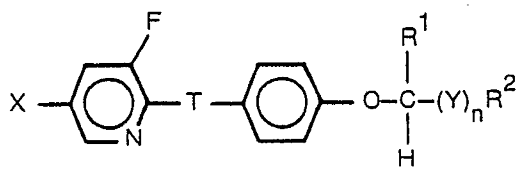

wherein X, T, Y, n, $R^1$ and $R^2$ are as hereinbefore defined which process comprises reacting a compound having the formula:

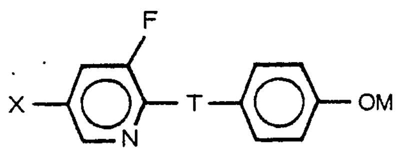

wherein M is H or a metallic cation and X and T are as hereinbefore defined with a compound having the formula:

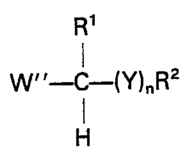

wherein W'' is Cl or Br and Y, n, $R^1$ and $R^2$ are as hereinbefore defined in the presence of a base.

The present invention further includes within its scope a process for the preparation of a compound of the formula:

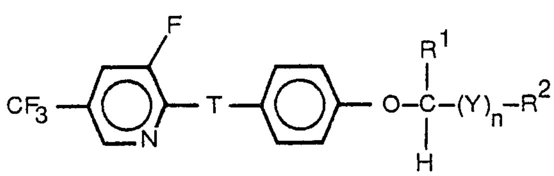

wherein T, Y, n, $R^1$ and $R^2$ are as hereinbefore defined which process comprises reacting a compound having the formula:

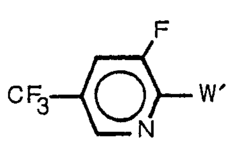

wherein W' is F, Cl or Br with a compound of the formula:

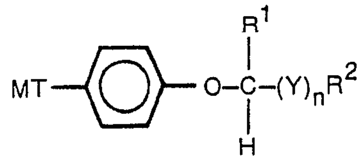

wherein M is hydrogen or a metallic cation, and T, Y, n, $R^1$ and $R^2$ are as hereinbefore defined in the presence of a base.

The present invention still further includes within its scope another process for the preparation of a compound of the formula:

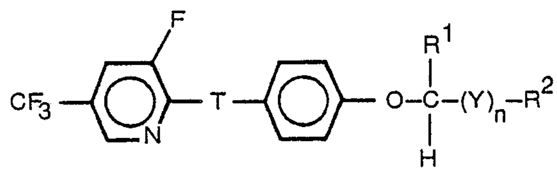

wherein T, Y, n, $R^1$ and $R^2$ are as hereinbefore defined which process comprises reacting a compound having the formula:

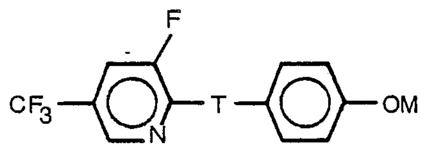

wherein M is H or a metallic cation and T is as hereinbefore defined with a compound having the formula:

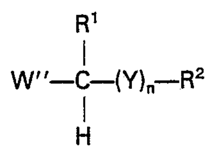

wherein W'' is Cl or Br and Y, n, $R^1$ and $R^2$ are as hereinbefore defined in the presence of a base.

The stereoisomers are readily separated as described in European 0002800 referred to above.

Certain of the pyridine reactants employed to make the novel pyridinyloxyphenoxy compounds of this invention are themselves novel compounds and such reactants may be made as generally described hereafter and as specifically set forth in the following examples or by methods analagous thereto, starting with known compounds.

We have unexpectedly found that the fluorine atom in 3-chloro-2-fluoro-5-(trifluoromethyl)pyridine can be readily replaced with a 2-cyano group by reacting said pyridine compound with an alkali metal cyanide, preferably potassium cyanide, in a polar aprotic solvent, preferably dimethylsulfoxide, at a temperature of 10°—50°C, preferably 20°—30°C. We have further found that the chlorine atom in the resulting product, i.e., 3-chloro-2-cyano-5-(trifluoromethyl)-pyridine is readily selectively replaced with fluorine by reaction with, e.g., caesium or potassium fluoride in a polar aprotic solvent, preferably dimethyl sulfoxide, at a temperature of 80°—140°C, preferably 90°—100°C. The cyano group in the resulting 2-cyano-3-fluoro-5-(trifluoromethyl)pyridine can be readily converted, by known procedures, to the corresponding acid or amide, as desired. The resulting acid may be readily converted to the corresponding bromine derivative by the Hunsdiecker reaction, as shown in the following Example 4, or the amide may be converted to the corresponding amine and then hydroxy compound by the Hoffmann hypobromite reaction followed by diazotization and replacement by hydroxide as known in the art. The latter is then treated with $POCl_3$ plus $PCl_3$, as known in the art, to prepare, for example, 2-chloro-3-fluoro-5-(trifluoromethyl)pyridine.

Alternatively, 2,3-difluoro-5-(trifluoromethyl)pyridine may be prepared by contacting 2,3-dichloro-5-(trifluoromethyl)pyridine or 3-chloro-2-fluoro-5-(trifluoromethyl)pyridine with a fluorinating agent and the 2,3-difluoro-5-(trifluoromethyl)pyridine may be used directly to make 2-(4-((3-fluoro-5-(trifluoromethyl)-2-pyridinyl)oxy)phenoxy)alkanoic acids and derivatives.

The following examples further illustrate the present invention. The preparative Examples relate to the preparation of intermediate compounds.

Preparation 1

Preparation of 3-chloro-2-cyano-5-(trifluoromethyl)pyridine

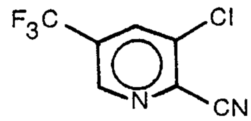

3-Chloro-2-fluoro-5-(trifluoromethyl)pyridine (obtained as a by-product from the fluorine exchange reaction when converting 2,3-dichloro-5-trifluoromethyl pyridine to 2,3-dichloro-5-(trifluoromethyl)-pyridine) (40.0 g, 0.2 mole) was put into 270 ml of dimethyl sulfoxide and stirred while potassium cyanide (14.4 g, 0.221 mole) was spooned in over a 20-minute period. The mixture was then stirred for another 20 minutes. The temperature was held between 23° and 28°C throughout the reaction. The mixture was poured into 600 ml of ice water and the product was extracted into hexane. The hexane was removed on a rotary evaporator. The product was treated with activated charcoal and distilled on a Vigreaux-Claisen still at 108°—110°C at 30 mm Hg to yield 31.45 g of colorless oil with an analysis of, in percent by weight:

| | | | |
|---|---|---|---|
| Calculated: | C = 40.70; | H = 0.98; | N = 13.56. |
| Found: | C = 40.42; | H = 0.99; | N = 13.66. |

Preparation 2

Preparation of 3-fluoro-2-cyano-5-(trifluoromethyl)pyridine

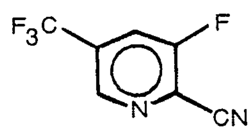

A flask fitted with an air stirrer and a takeoff head was set up. Caesium fluoride (45.6 g, 0.3 mole), potassium carbonate (1.2 g) and 350 ml of dimethyl sulfoxide were put into the flask and heated and stirred under vacuum (30 mm). 120 Ml of dimethyl sulfoxide was distilled off to dry the system. The reaction mixture was cooled to 80°C, the vacuum was released and 3-chloro-2-cyano-5-(trifluoromethyl)pyridine (41.6 g—0.201 mole) was added over a 7-minute period. The reaction mixture was then warmed to 93°C and held at 93°—111°C for about 20 minutes. The mixture was then cooled to 54°C, poured over ice and extracted twice with hexane and once with methylene chloride. The solvents were removed and the product was distilled at about 30 mm Hg at 90°—94°C to yield 29.6 g of colorless oil which had an analysis of, in percent by weight:

| | | | |
|---|---|---|---|
| Calculated: | C = 44.22; | H = 1.06; | N = 14.74. |
| Found: | C = 43.53; | H = 1.11; | N = 14.44. |

Preparation 3

Preparation of 3-fluoro-5-(trifluoromethyl)picolinic acid

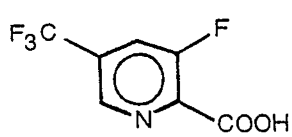

2-Cyano-3-fluoro-5-(trifluoromethyl)-pyridine (11.1 g, 0.0584 mole) was put into 87 ml of 90% sulfuric acid in a beaker. The mixture was stirred and heated at 100°—112°C for 1¼ hours. The reaction mixture was then cooled, poured over ice and the solids that came down were filtered off. The solids were dissolved in a dilute solution of NaOH. Any material that didn't go into solution was filtered out and the filtrate was acidified with aqueous HCl and the precipitate was filtered off and dried. This yielded 6.47 g of solid product with an analysis of, in percent by weight:

| | | | |
|---|---|---|---|
| Calculated: | C = 40.20; | H = 1.45; | N = 6.70. |
| Found: | C = 39.29; | H = 1.35; | N = 6.98. |

1.05 g of a second crop of solids was obtained on standing which exhibited the same IR spectrum as the first solids out. These were combined and used to make the following bromo compound.

Preparation 4

Preparation of 2-bromo-3-fluoro-5-(trifluoromethyl)pyridine

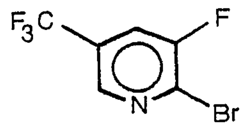

The 3-fluoro-5-(trifluoromethyl)-picolinic acid (7.35 g, 0.035 mole) starting material was put into 150 ml of dry carbon tetrachloride and then red mercuric oxide (9.1 g, 0.042 mole) was added and the mixture was stirred and refluxed 1 hour and 25 minutes. A solution of bromine (6.7 g, 0.042 mole) in 20 ml of dry carbon tetrachloride was added slowly with the mixture at reflux over the next 2⅓ hours. Light from a UV lamp was directed on the reaction mixture during the addition and the reaction mixture was refluxed for another hour. 25 Ml more of dry carbon tetrachloride was added and the refluxing was continued for about 16 hours more while UV radiation was applied. The reaction was then filtered through CELITE diatomaceous earth to remove the mercury salt. The carbon tetrachloride was removed on a still and the product was distilled over to yield 2.45 g of yellow oil with an analysis of, in percent by weight:

| | | | |
|---|---|---|---|
| Calculated: | C = 29.53; | H = 0.83; | N = 5.74. |
| Found: | C = 29.36; | H = 0.77; | N = 5.82. |

The gas chromatograph showed the oil to be a 99+% pure compound. CELITE is a Registered Trade Mark.

Example 1

Preparation of 2-(4-(3-fluoro-5-(trifluoromethyl)-2-pyridinyl-oxy)phenoxy)propanoic acid

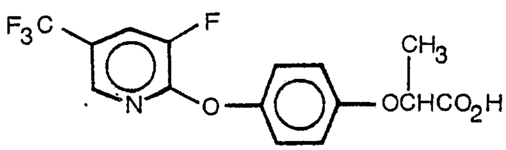

2-(4-Hydroxyphenoxy) propanoic acid (1.80 g, 0.00988 mole) was dissolved in 15 ml of dimethyl sulfoxide. A solution of sodium hydroxide (0.8 g, 0.02 mole in 1 ml of water) was added and the reaction mixture was heated under nitrogen to 48°C over a 27 minute period. 2-Bromo-3-fluoro-5-(trifluoromethyl)-pyridine (2.40 g, 0.00984 mole) dissolved in 5 ml of dimethyl sulfoxide was added and the reaction mixture was heated at 75°—78°C for 40 minutes. The mixture was poured into 150 ml of cold water and acidified with aqueous HCl. A gum came down. Upon work-up and purification, a fraction (0.45 g) was obtained which had an elemental analysis of, in percent by weight.

| | | | |
|---|---|---|---|
| Calculated: | C = 52.18; | H = 3.21; | N = 4.06. |
| Found: | C = 51.89; | H = 3.19; | N = 4.02. |

This material had a melting point of 130°—132°C.

Example 2

Preparation of 2-(4-(3-fluoro-5-trifluoromethyl)-2-pyridinyl-oxy)phenoxy)propionamide

A. A fresh sample of 2-(4-(3-fluoro-5-(trifluoromethyl)-2-pyridinyl-oxy)phenoxy propanoic acid was prepared by the reaction of 2,3-difluoro-5-(trifluoromethyl)pyridine with 2-(4-hydroxyphenoxy)propanoic acid in the presence of 2 moles of sodium hydroxide as above described and 9.0 g (0.026 mole) was refluxed in excess $SOCl_2$ for about ½ hour to prepare the corresponding acid chloride. The excess $SOCl_2$ was removed by heating to 115°C under an aspirator vacuum. The resulting acid chloride was added to a mixture of concentrated ammonium hydroxide (30 ml) and methanol (60 ml). The flask was rinsed with an additional 15 ml ammonium hydroxide mixed with about 25 ml of methanol and the two fractions were combined. The acid amide formed as a solid which was filtered out, rinsed with water, slurried in water, filtered, dried and analyzed. M.P. 140°—141°C.

| Analysis: | %C | %H | %N |
|---|---|---|---|
| Calculated: | 52.33 | 3.51 | 8.14 |
| Found: | 52.54 | 3.46 | 8.09 |

B. Another propionamide of the invention, as set forth below, was prepared:

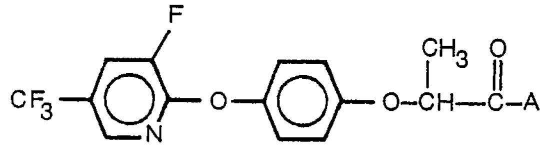

| A | M.P.°C | | Analysis | | | |
|---|---|---|---|---|---|---|
| | | | %C | %H | %N | %Cl |
| —NH—Cl | 142—143.5 | Calc.: | 55.45 | 3.32 | 6.16 | 7.80 |
| | | Found: | 55.88 | 3.34 | 6.11 | 7.70 |

## Example 3

Preparation of 2-(4-(3-fluoro-5-(trifluoromethyl)-2-pyridinyl-oxy)phenoxy)propionitrile

A portion of the amide prepared in Example 2A (5.68 g, 0.0165 mole) was refluxed with an excess of $POCl_3$ for about 2 hours after which the excess $POCl_3$ was removed by distillation under an aspirator vacuum. The reaction mixture was poured over ice and extracted with methylene chloride. The methylene chloride was removed on a rotary evaporator and the crude material was taken up in hexane and decolorized with charcoal. A gummy material formed which was again placed on the evaporator leaving an oil which was recovered and analyzed.

| Analysis: | %C | %H | %N |
|---|---|---|---|
| Calculated: | 55.22 | 3.09 | 8.59 |
| Found: | 55.64 | 3.00 | 8.81 |

Refractive Index = 1.5067 at 25°C.

## Example 4

Preparation of 2-(4-(3-flùoro-5-(trifluoromethyl)-2-pyridinyl-oxy)phenoxy)propanoic acid methyl ester

Following the procedure of Example 2A the acid chloride was prepared by refluxing a portion of the acid from Example 2A with thionyl chloride. The resulting acid chloride (3.45 g, 0.01 mole) was then reacted with methanol (1.0 g, 0.0312 mole) in the presence of triethylamine (2.0 g, 0.02 mole) in 20 ml of toluene at 80°—88°C. The salt was removed by filtration, then rinsed with hexane, the filtrate combined and solvent removed by evaporation. The crude product was taken up in hexane, some solids removed by filtration and the solution decolorized with charcoal after which solvents were removed under vacuum. The product gradually solidified and was recovered and analyzed. M.P. 50°—52°C.

| Analysis: | %C | %H | %N |
|---|---|---|---|
| Calculated: | 53.49 | 3.63 | 3.90 |
| Found: | 53.82 | 3.68 | 3.87 |

Following the above procedure except to employ other alcohols or thioalcohols as the esterifying agent the following compounds were prepared having the general formula:

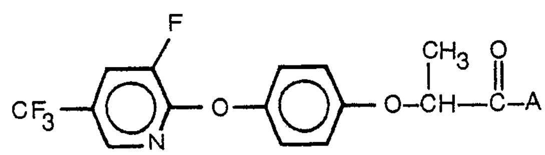

| A | Prep. Properties | | C | Percentage Compositions H | N | S |
|---|---|---|---|---|---|---|
| $—OC_4H_9$ | Oil | Calc.: | 56.86 | 4.77 | 3.49 | |
| | | Found: | 56.85 | 4.77 | 3.51 | |
| $—OCH_2CH_2OCH_2CH_3$ | Oil, R.I. = 1.4944 @ 25°C | Calc.: | 54.68 | 4.59 | 3.36 | |
| | | Found: | 54.74 | 4.44 | 3.42 | |
| $—SC_4H_9$ | Oil, R.I. = 1.5164 @ 25°C | Calc.: | 54.67 | 4.59 | 3.36 | 7.68 |
| | | Found: | 54.87 | 4.54 | 3.39 | 7.59 |

R.I. = refractive index corrected to 25°C.

Example 5

Preparation of 2-(4-(3-fluoro-5-(trifluoromethyl)-2-pyridinyl-oxy)phenoxy-1-propanol

The methyl ester of Example 4 (5.7 g, 0.0159 mole) was dissolved in methanol (75 ml) and a solution of sodium borohydride (3.5 g, 0.0954 mole) was added dropwise to the cooled (19°C) solution, maintaining the temperature at about 25°C. The mixture was stirred for about 1½ hours after which the temperature was down to 18°C. On standing 30 minutes, the temperature increased to 23.5°C after which the reaction mixture was warmed to 42°C (30 minutes), continued stirring without heat for 30 minutes, poured into a beaker and added ice water (200 ml). Extracted with hexane and then twice with methylene chloride. Combined extracts, removed solvents and obtained 4.94 g of the above indicated product as a light yellow oil. R.I. = 1.5144 @ 25°C.

| Analysis: | %C | %H | %N |
|---|---|---|---|
| Calculated: | 54.38 | 3.96 | 4.23 |
| Found: | 54.25 | 3.98 | 4.44 |

Example 6

Preparation of the R-enantiomer of 2-(4-(3-fluoro-5-(trifluoromethyl)-2-pyridinyl-oxy)phenoxy)propanoic acid-methyl ester

A mixture of 2.11 g (7.72 mmol) of 4-(3-fluoro-5-(trifluoromethyl))-2-pyridyloxyphenol (prepared as in preparation 5) 1.07 g (7.72 mmol) of anhydrous $K_2CO_3$ and 14.1 g (77.2 mmol) of the methane sulfonate of the methyl ester of L-(+)-lactic acid in 16 ml of dry dimethylsulfoxide (DMSO) was stirred at room temperature for 43 hours. The reaction mixture was partitioned between diethylether and water. The organic phase was separated, dried ($Na_2SO_4$) and evaporated at reduced pressure to give a colorless liquid. Purification by high pressure liquid chromatography on silica gel eluting with ethyl acetate-hexane (3:22, v/v) gave 2.15 g (78%) of the R-enantiomer of the desired product as a colorless oil: $[\alpha]_D^{25}$ + 31.4° ($CHCl_3$, C 0.0110 g/ml); IR ($CCl_4$) 1766 and 1741 $cm^{-1}$; $^1H$ NMR ($CDCl_3$) δ 8.0—8.2 (1H, m), 7.5—7.8 (1H, m), 6.7—7.2 (4H, m), 4.71 (1H, q), 3.73 (3H, s) and 1.59 (3H, d); $^{19}F$ NMR ($CDCl_3$, ppm upfield from $C_6F_6$) 102.1 (s) and 26.3 (d) *Anal.* Calculated for $C_{16}H_{13}F_4NO_4$: C, 53.49; H, 3.65; N, 3.90. Found: C, 53.61; H, 3.53; N, 3.86. The optical purity of the sample was determined to be ≥90% ee by $^1H$ NMR analysis in the presence of Eu(tfc)$_3$.

Preparation 5

Preparation of 4-(3-fluoro-5-(trifluoromethyl)-2-pyridinyl-oxy)phenol

Hydroquinone (4.4 g, 0.04 mole) was dissolved in 65 ml of dimethylsulfoxide and powdered sodium hydroxide (1.4 g, 0.035 mole) was added in one portion and the mixture stirred under nitrogen atmosphere for 10 minutes to convert to the sodium salt. 2,3-Difluoro-5-(trifluoromethyl)pyridine (6.0 g, 0.033 mole) was then added and the mixture stirred at 50°C for 1.5 hours then warmed to 60°C for a moment, let cool for 15 minutes and then poured into 500 ml of cold water. Additional sodium hydroxide (~3 g in water) was added to convert the desired product to its sodium salt. The insoluble bis-derivative was then removed by extraction with hexane. The clear aqueous phase was separated, cooled and acidified with concentrated hydrochloric acid. The solid which separated was collected on a filter, washed, dried on a vacuum funnel and taken up in hot hexane. After treating with decolorizing carbon, filtering, concentrating and cooling the white crystalline product separated. Yield: 2.6 g. M.P. 97.5—98.5.

| Analysis: | %C | %H | %N |
|---|---|---|---|
| Calculated: | 52.76 | 2.58 | 5.12 |
| Found: | 52.71 | 2.57 | 5.12 |

The compounds of the present invention have been found to be suitable for use in methods for the selective pre- and postemergent control of annual and perennial grassy weeds. These compounds, the active ingredients of the present invention, have been found to have advantage over prior art compounds in the control of annual and perennial grassy weeds in that the present compounds control such weeds at substantially lower dosage rates. In addition, the present compounds are sufficiently tolerant towards most broad leafed crops to contemplate control of grassy weeds therein at substantially commercially practicable levels, particularly so with the preferred compounds. In addition, certain of the compounds have sufficient tolerance towards cereal crops such as wheat to enable selective grassy weed control in these crops as well.

For such uses, unmodified active ingredients of the present invention can be employed. However, the present invention embraces the use of the compounds in composition form with an inert material, known in the art as an agricultural adjuvant or carrier, in solid or liquid form. Thus, for example, an active ingredient can be dispersed on a finely-divided solid and employed therein as a dust or granule. Also, the active ingredients, as liquid concentrates or solid compositions comprising one or more of the active ingredients can be dispersed in water, typically with aid of a wetting agent, and the resulting aqueous dispersion employed as a spray. In other procedures, the active ingredients can be employed as a constituent of organic liquid compositions, oil-in-water and water-in-oil emulsions or water dispersions,

with or without the addition of wetting, dispersing, or emulsifying agents. Suitable adjuvants of the foregoing type are well known to those skilled in the art.

The herbicidally effective concentration of the active ingredients in solid or liquid compositions generally is from 0.0003 to 95 percent by weight or more. Concentrations from 0.05 to 50 percent by weight are often employed. In compositions to be employed as concentrates, the active ingredient can be present in a concentration from 5 to 98 weight percent. The active ingredient compositions can also contain other compatible additaments, for example, phytotoxicants, plant growth regulants and other biologically active compounds used in agriculture.

In further embodiments, the compounds of the present invention or compositions containing the same, can be advantageously employed in combination with one or more additional pesticidal compounds. Such additional pesticidal compounds may be insecticides, nematocides, miticides, arthropodicides, herbicides, fungicides or bactericides that are compatible with the compounds of the present invention in the medium selected for application and not antagonistic to the activity of the present compounds. Accordingly, in such embodiments, the pesticidal compound is employed as a supplemental toxicant for the same or for a different pesticidal use or as an additament. The compounds in combination can generally be present in a ratio of from 1 to 100 parts of the compound of the present invention with from 100 to 1 parts of the additional compound(s).

The active ingredients of the present invention have been found to possess desirable herbicidal activity in general against grassy weeds such as foxtail, barnyardgrass, wild oats, seedling johnsongrass and crabgrass in preemergent operations and also against the same grasses in postemergent operations while being tolerant to important broadleaf crops such as cotton, soybeans, sugarbeets and rape and in the case of certain of the compounds, certain cereal crops such as wheat. These compounds are also uniquely effective in selectively controlling perennial grassy weeds such as johnsongrass, quackgrass, bermudagrass and dallisgrass.

The active ingredients of the present invention have been found to possess particularly desirable herbicidal activity against wild oats, foxtail, barnyardgrass, crabgrass and seedling johnsongrass in postemergent operations as well as desirable broad spectrum activity against the perennial grassy weeds listed above and at lower dosage rates than the substituted propanoates and propanols of the prior art while showing high selectivity to broadleaf crops and, in the case of certain of the compounds, wheat.

The present compounds which are substituted propanols or propyl ethers are more effective in preemergent operations than in postemergent applications.

The exact rate to be applied is dependent not only on a specific active ingredient being applied, but also on a particular action desired, the plant species to be modified and the stage of growth thereof as well as the part of the plant to be contacted with the toxic active ingredient. Thus, all of the active ingredients of the present invention and compositions containing the same may not be equally effective at similar concentrations or against the same plant species.

In postemergent operations a dosage of about 0.05 to about 20 pounds/acre (0.056—22.4 kg/hectare) is generally applicable, although not all compounds are equally effective and some weeds are more difficult to control. Thus, a dosage rate in the range of about 0.01 to about 1.0 pound/acre (0.01—1.12 kg/hectare) is preferred in postemergent control of annual grassy weeds, while about 0.05 to about 5 pounds/acre (0.056—5.6 kg/hectare) is a preferred dosage range for the postemergent control of perennial grassy weeds. In applications to tolerant crops a weed controlling but less than crop damaging amount of from about .005 to about 1.0 lb/acre (0.0056 to 1.12 kgs/hectare) is generally employed.

In preemergent operations a dosage rate of 0.01 to 10 lbs/acre (0.011 to 11.2 kgs/hectare), preferably 0.05 to 2.0 lbs/acre (0.056 to 2.25 kgs/hectare) and most preferably 0.1 to 1 lb/acre (0.11 to 1.12 kgs/hectare) is generally employed.

The following examples illustrate effects of the compounds of this invention.

## Example 7

In representative operations, each compound to be utilized in a series of tests is dissolved in acetone to one-half of the final volume (twice the final concentration) to be used and the acetone solution in each case is admixed with an equal volume of water containing 0.1 percent by weight of the non-ionic surfactant TWEEN® 20 (a polyoxyethylene sorbitan monolaurate). The compositions, generally in the nature of an emulsion, were employed to spray separate respective plant species which had been grown to a height of 2—6 inches in soil of good nutrient content in a greenhouse. Sufficient amounts were employed to provide various application rates as listed in the table. The various beds were positioned side by side and exposed to substantially identical conditions of temperature and light. Each bed was maintained so as to prevent any interaction with test compounds in different seed beds. Other portions of the plants were left untreated to serve as controls. After treatment, the plants were maintained for about two weeks under greenhouse conditions conducive for good plant growth and watered as necessary. The specific plant species, test compound and dosage and the percent postemergent control obtained are set forth in the table below. Control refers to the reduction in growth compared to the observed results of the same untreated species. Note the "NT" means "not tested".

Plant species in these tests were the following:

| Common Name | Scientific Name |
|---|---|
| Barnyardgrass (Watergrass) | *Echinochloa crusgalli* |
| Crabgrass | *Digitaria sanquinalis* |
| Yellow foxtail | *Setaria lutescens* |
| Johnsongrass | *Sorghum halepense* |
| Wild Oats | *Avena fatua* |
| Cotton | *Gossypium hirsutum* |
| Rape | *Brassica napus* |
| Soybeans | *Glycine max* |
| Sugarbeet | *Beta vulgaris* |
| Wheat | *Triticum aestivum* |

POSTEMERGENT CONTROL OF PLANT SPECIES

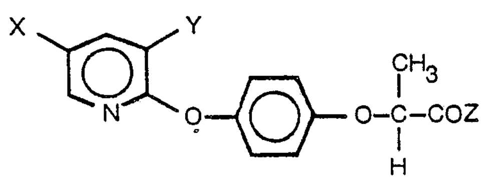

| Compound Tested | | | Plant Species | Dosage in ppm | | | | | |
|---|---|---|---|---|---|---|---|---|---|
| X | Y | Z | | 125 | 62.5 | 31.25 | 15.6 | 7.8 | 3.9 |
| $CF_3$ | F | OH | Cotton | 0 | 0 | 0 | 0 | 0 | 0 |
| | | | Rape | 0 | 0 | 0 | 0 | 0 | 0 |
| | | | Soybean | 0 | 0 | 0 | 0 | 0 | 0 |
| | | | Sugarbeet | 0 | 0 | 0 | 0 | 0 | 0 |
| | | | Barnyardgrass | 100 | 100 | 100 | 80 | 30 | 0 |
| | | | Crabgrass | 100 | 100 | 100 | 90 | 80 | 15 |
| | | | Yellow Foxtail | 100 | 100 | 100 | 65 | 20 | 0 |
| | | | Johnson grass | 100 | 100 | 100 | 100 | 50 | 0 |
| | | | Wild Oats | 100 | 100 | 80 | 40 | 0 | 0 |
| | | | Wheat | 100 | 100 | 90 | 40 | 0 | NT |

POSTEMERGENT CONTROL OF PLANT SPECIES

| Compound Tested | | | | Dosage in ppm | | | | | |
|---|---|---|---|---|---|---|---|---|---|
| X | Y | Z | Plant Species | 125 | 62.5 | 31.25 | 15.6 | 7.8 | 3.9 |
| $CF_3$ | F | $OCH_3$ | Cotton | 0 | 0 | 0 | 0 | 0 | 0 |
| (R enantiomer- | | | Rape | 0 | 0 | 0 | 0 | 0 | 0 |
| 90% optical | | | Soybean | 0 | 0 | 0 | 0 | 0 | 0 |
| purity) | | | Sugarbeets | 0 | 0 | 0 | 0 | 0 | 0 |
| | | | Barnyardgrass | 100 | 100 | 100 | 100 | 95 | 10 |
| | | | Crabgrass | 100 | 100 | 100 | 100 | 100 | 90 |
| | | | Yellow Foxtail | 100 | 100 | 95 | 90 | 40 | 0 |
| | | | Johnson grass | 100 | 100 | 100 | 100 | 70 | 70 |
| | | | Wild Oats | 100 | 100 | 100 | 100 | 0 | 0 |
| | | | Wheat | 90 | 100 | 100 | 55 | 65 | 0 |
| $CF_3$ | F | $OCH_3$ | Cotton | 0 | 0 | 0 | 0 | 0 | 0 |
| | | | Rape | 0 | 0 | 0 | 0 | 0 | 0 |
| | | | Soybean | 0 | 0 | 0 | 0 | 0 | 0 |
| | | | Sugarbeets | 0 | 0 | 0 | 0 | 0 | 0 |
| | | | Barnyardgrass | 100 | 100 | 100 | 100 | 10 | 0 |
| | | | Crabgrass | 100 | 100 | 100 | 100 | 90 | 50 |
| | | | Yellow Foxtail | 100 | 100 | 40 | 50 | 10 | 0 |
| | | | Johnson grass | 100 | 100 | 100 | 90 | 90 | 50 |
| | | | Wild Oats | 100 | 100 | 90 | 20 | 0 | 0 |
| | | | Wheat | 100 | 100 | 20 | 0 | NT | NT |
| $CF_3$ | F | $OC_2H_4$- | Cotton | 0 | 0 | 0 | 0 | 0 | 0 |
| | | $OC_2H_5$ | Rape | 0 | 0 | 0 | 0 | 0 | 0 |
| | | | Soybean | 0 | 0 | 0 | 0 | 0 | 0 |
| | | | Sugarbeets | 0 | 0 | 0 | 0 | 0 | 0 |
| | | | Barnyardgrass | 100 | 100 | 100 | 100 | 90 | 70 |
| | | | Crabgrass | 100 | 100 | 100 | 100 | 100 | 90 |
| | | | Yellow Foxtail | 100 | 100 | 100 | 100 | 85 | 10 |
| | | | Johnson grass | 100 | 100 | 100 | 100 | 100 | 90 |
| | | | Wild Oats | 100 | 100 | 90 | 80 | 40 | 0 |
| | | | Wheat | 100 | 100 | 90 | 85 | 40 | 0 |

POSTEMERGENT CONTROL OF PLANT SPECIES

| Compound Tested | | | | Dosage in ppm | | | | | |
|---|---|---|---|---|---|---|---|---|---|
| X | Y | Z | Plant Species | 125 | 62.5 | 31.25 | 15.6 | 7.8 | 3.9 |
| $CF_3$ | F | NH-Cl | Cotton | 0 | 0 | 0 | 0 | 0 | 0 |
| | | | Rape | 0 | 0 | 0 | 0 | 0 | 0 |
| | | | Soybean | 0 | 0 | 0 | 0 | 0 | 0 |
| | | | Sugarbeets | 0 | 0 | 0 | 0 | 0 | 0 |
| | | | Barnyardgrass | 100 | 100 | 90 | 70 | 20 | 0 |
| | | | Crabgrass | 100 | 100 | 100 | 100 | 90 | 40 |
| | | | Yellow Foxtail | 100 | 100 | 100 | 100 | 80 | 10 |
| | | | Johnson grass | 100 | 100 | 100 | 100 | 95 | 80 |
| | | | Wild Oats | 100 | 100 | 30 | 50 | 20 | 0 |
| | | | Wheat | 100 | 100 | 80 | 40 | 10 | 0 |
| $CF_3$ | F | $SC_4H_9$ | Cotton | 0 | 0 | 0 | 0 | 0 | 0 |
| | | | Rape | 0 | 0 | 0 | 0 | 0 | 0 |
| | | | Soybean | 0 | 0 | 0 | 0 | 0 | 0 |
| | | | Sugarbeets | 0 | 0 | 0 | 0 | 0 | 0 |
| | | | Barnyardgrass | 100 | 100 | 100 | 70 | 80 | 40 |
| | | | Crabgrass | 100 | 100 | 100 | 100 | 100 | 90 |
| | | | Yellow Foxtail | 100 | 100 | 100 | 100 | 95 | 65 |
| | | | Johnson grass | 100 | 100 | 100 | 95 | 100 | 75 |
| | | | Wild Oats | 100 | 100 | 90 | 10 | 0 | NT |
| | | | Wheat | 100 | 100 | 90 | 80 | 10 | 0 |
| $CF_3$ | F | $NH_2$ | Cotton | 0 | 0 | 0 | 0 | 0 | 0 |
| | | | Rape | 0 | 0 | 0 | 0 | 0 | 0 |
| | | | Soybean | 0 | 0 | 0 | 0 | 0 | 0 |
| | | | Sugarbeets | 0 | 0 | 0 | 0 | 0 | 0 |
| | | | Barnyardgrass | 100 | 100 | 100 | 95 | 95 | 20 |
| | | | Crabgrass | 100 | 100 | 100 | 100 | 100 | 90 |
| | | | Yellow Foxtail | 100 | 100 | 100 | 100 | 90 | 15 |
| | | | Johnson grass | 100 | 100 | 100 | 100 | 95 | 90 |
| | | | Wild Oats | 100 | 100 | 90 | 80 | 70 | 0 |
| | | | Wheat | 100 | 100 | 90 | 80 | 75 | 50 |

Example 8

So as to clearly illustrate the phytotoxic properties of the various active ingredients of the present invention applied preemergently, a controlled greenhouse experiment is described below.

The seeds of various species of plants were plated in beds of good agricultural soil in a greenhouse. A number of compositions of the present invention, generally in the nature of an aqueous emulsion, were applied at rates listed in the table so as to deposit a predetermined amount of active ingredients uniformly throughout the surface of the bed. Another seed bed was treated only with water to serve as a control. After treatment the seed beds were maintained for two weeks under greenhouse conditions conducive for good plant growth and watered as necessary. The specific plant species, test compound, and dosage and the percent preemergent control are set forth in the table below. Control refers to the reduction in growth compared to the observed results of the same untreated species.

PREEMERGENT CONTROL OF PLANT SPECIES

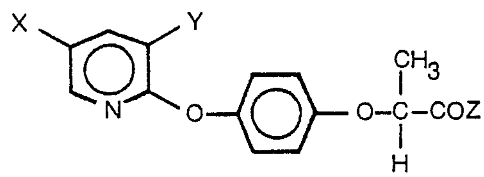

| Compound Tested | | | | Dosage (Kg/Hectare) | | | | |
|---|---|---|---|---|---|---|---|---|
| X | Y | Z | Plant Species | .28 | .14 | .07 | .035 | .018 |
| $CF_3$ | F | OH | Cotton | 0 | 0 | 0 | 0 | 0 |
| | | | Rape | 0 | 0 | 0 | 0 | 0 |
| | | | Soybean | 0 | 0 | 0 | 0 | 0 |
| | | | Sugarbeet | 0 | 0 | 0 | 0 | 0 |
| | | | Barnyardgrass | 100 | 100 | 97 | 85 | 10 |
| | | | Crabgrass | 100 | 100 | 100 | 60 | 5 |
| | | | Yellow Foxtail | 100 | 100 | 85 | 30 | 10 |
| | | | Johnson grass | 100 | 98 | 97 | 65 | 25 |
| | | | Wild Oats | 100 | 99 | 80 | 65 | 10 |
| | | | Wheat | 100 | 97 | 40 | 20 | 0 |

PREEMERGENT CONTROL OF PLANT SPECIES

| Compound Tested | | | Plant Species | Dosage (Kg/Hectare) | | | | |
|---|---|---|---|---|---|---|---|---|
| X | Y | Z | | .28 | .14 | .07 | .035 | .018 |
| $CF_3$ (R enantionmer — ≥ 90% optical purity) | F | $OCH_3$ | Cotton | 0 | 0 | 0 | 0 | 0 |
| | | | Rape | 0 | 0 | 0 | 0 | 0 |
| | | | Soybean | 0 | 0 | 0 | 0 | 0 |
| | | | Sugarbeet | 0 | 0 | 0 | 0 | 0 |
| | | | Barnyardgrass | 100 | 100 | 100 | 95 | 50 |
| | | | Crabgrass | 100 | 100 | 100 | 100 | 100 |
| | | | Yellow Foxtail | 100 | 100 | 100 | 90 | 70 |
| | | | Johnson grass | 100 | 100 | 100 | 100 | 90 |
| | | | Wild Oats | 100 | 100 | 98 | 40 | 30 |
| | | | Wheat | 100 | 100 | 100 | 90 | 40 |
| $CF_3$ | F | $OCH_3$ | Barnyardgrass | 100 | 100 | 80 | 30 | 10 |
| | | | Crabgrass | 100 | 100 | 100 | 100 | 90 |
| | | | Yellow Foxtail | 100 | 100 | 50 | 40 | 10 |
| | | | Johnson grass | 100 | 100 | 100 | 90 | 50 |
| | | | Wild Oats | 100 | 100 | 95 | 30 | 0 |
| | | | Wheat | 100 | 100 | 98 | 40 | 10 |

At 0.28 kg/hectare there was no damage to cotton, rape, soybeans or sugarbeets

PREEMERGENT CONTROL OF PLANT SPECIES

| Compound Tested | | | | Dosage (Kg/Hectare) | | | | |
|---|---|---|---|---|---|---|---|---|
| X | Y | Z | Plant Species | .28 | .14 | .07 | .035 | .018 |
| $CF_3$ | F | $OC_4H_9$ | Barnyardgrass | NT | 100 | 90 | 50 | 0 |
| | | | Crabgrass | NT | 100 | 100 | 100 | 40 |
| | | | Yellow Foxtail | NT | 100 | 100 | 30 | 0 |
| | | | Johnson grass | NT | 100 | 100 | 100 | 20 |
| | | | Wild Oats | NT | 40 | 30 | 0 | 0 |
| | | | Wheat | NT | 100 | 100 | 60 | 10 |
| | | | At 0.28 kg/hectare there was no damage to cotton, rape, soybeans or sugarbeets | | | | | |
| $CF_3$ | F | $NH_2$ | Barnyardgrass | 100 | 100 | 70 | 20 | 0 |
| | | | Crabgrass | 100 | 100 | 100 | 100 | 30 |
| | | | Yellow Foxtail | 100 | 100 | 100 | 30 | 0 |
| | | | Johnson grass | 100 | 100 | 80 | 60 | 10 |
| | | | Wild Oats | 100 | 100 | 100 | 30 | 0 |
| | | | Wheat | 100 | 100 | 97 | 70 | 0 |

At 0.28 kg/hectare (0.25 lb/acre) there was no damage to cotton, rape, soybeans or sugarbeets

PREEMERGENT CONTROL OF PLANT SPECIES

| Compound Tested | | | | Dosage (Kg/Hectare) | | | | |
|---|---|---|---|---|---|---|---|---|
| X | Y | Z | Plant Species | .28 | .14 | .07 | .035 | .018 |
| $CF_3$ | F | H<br>N Cl | Barnyardgrass | 100 | 100 | 70 | 20 | 0 |
| | | | Crabgrass | 100 | 100 | 100 | 100 | 40 |
| | | | Yellow Foxtail | 100 | 100 | 100 | 95 | 30 |
| | | | Johnson grass | 100 | 100 | 100 | 30 | 0 |
| | | | Wild Oats | 100 | 100 | 60 | 20 | 0 |
| | | | Wheat | 100 | 50 | 30 | 0 | NT |

At 0.28 kg/hectare there was no damage to cotton, rape, soybeans or sugarbeets

| | | | | | | | | |
|---|---|---|---|---|---|---|---|---|
| $CF_3$ | F | $SC_4H_9$ | Barnyardgrass | 100 | 100 | 100 | 30 | 0 |
| | | | Crabgrass | 100 | 100 | 100 | 100 | 100 |
| | | | Yellow Foxtail | 100 | 100 | 100 | 30 | 0 |
| | | | Johnson grass | 100 | 100 | 90 | 80 | 30 |
| | | | Wild Oats | 100 | 100 | 100 | 70 | 30 |
| | | | Wheat | 100 | 100 | 100 | 70 | 20 |

At 0.28 kg/hectare there was no damage to cotton, rape, soybeans or sugarbeets

| | | | | | | | | |
|---|---|---|---|---|---|---|---|---|
| $CF_3$<br>*replaces COZ | F | CN* | Barnyardgrass | 100 | 100 | 80 | 50 | 10 |
| | | | Crabgrass | 100 | 100 | 100 | 100 | 30 |
| | | | Yellow Foxtail | 100 | 100 | 90 | 90 | 30 |
| | | | Johnson grass | 100 | 100 | 95 | 60 | 20 |
| | | | Wild Oats | NT | 100 | 100 | 20 | 0 |
| | | | Wheat | 100 | 30 | 40 | 0 | 0 |

At 0.28 kg/hectare there was no damage to cotton, rape, soybeans or sugarbeets

PREEMERGENT CONTROL OF PLANT SPECIES

| Compound Tested | | | | Dosage (Kg/Hectare) | | | | |
|---|---|---|---|---|---|---|---|---|
| X | Y | Z | Plant Species | .28 | .14 | .07 | .035 | .018 |
| $CF_3$ | F | $OC_2H_4OC_2H_5$ | Barnyardgrass | 100 | 100 | 100 | 20 | 0 |
| | | | Crabgrass | 100 | 100 | 100 | 50 | 10 |
| | | | Yellow Foxtail | 100 | 100 | 90 | 20 | 0 |
| | | | Johnson grass | 100 | 90 | 80 | 20 | 0 |
| | | | Wild Oats | 100 | 100 | 70 | 30 | 0 |
| | | | Wheat | 100 | 100 | 100 | 50 | 20 |
| | | | At 0.28 kg/hectare there was no damage to cotton, rape, soybeans or sugarbeets | | | | | |
| $CF_3$ *replaces Radical COZ | F | $CH_2OH$* | Barnyardgrass | 100 | 100 | 100 | 70 | 40 |
| | | | Crabgrass | 100 | 100 | 100 | 100 | 100 |
| | | | Yellow Foxtail | 100 | 100 | 100 | 100 | 50 |
| | | | Johnson grass | 100 | 90 | 80 | 20 | 0 |
| | | | Wild Oats | 100 | 100 | 40 | 10 | 0 |
| | | | Wheat | 100 | 100 | 100 | 50 | 40 |
| | | | At 0.28 kg/hectare there was no damage to cotton, rape, soybeans or sugarbeets | | | | | |

Other compounds within the scope of the present invention, e.g., the various metal salts, amine salts and other derivatives of the above described compounds may also be employed to control certain plant species with results commensurate to the above described results.

**Claims for the Contracting States: BE CH DE FR IT LI NL SE**

1. A herbicidally active compound or a resolved optically active isomer thereof having the formula:

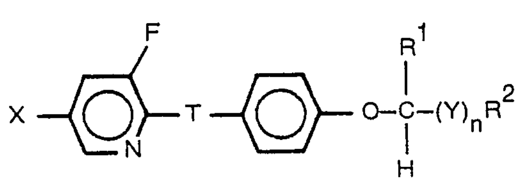

wherein
X is $CF_3$, $CHF_2$ or $CClF_2$;
T is O or S;
Y is an alkyl, alkenyl or alkynyl group containing an even number of from 2 to 18 carbon atoms;
n is 0 or 1;
$R^1$ is a $C_1$—$C_3$ alkyl group; and
$R^2$ is a carboxyl group; an alkali, alkaline earth metal, ammonium or organic amine salt thereof; or an organic group that can be hydrolyzed and/or oxidized in plants or the soil to a carboxyl group that is in undissociated and/or dissociated form.

2. A herbicidally active compound or a resolved optically active isomer thereof having the formula:

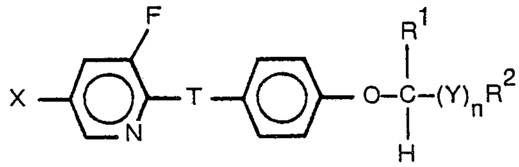

wherein

X is $CF_3$, $CHF_2$ or $CClF_2$;

T is O or S;

Y is an alkyl, alkenyl or alkynyl group containing an even number of from 2 to 18 carbon atoms;

n is 0 or 1;

$R^1$ is a $C_1$—$C_3$ alkyl group; and

$R^2$ is a carboxyl group; an alkali, alkaline earth metal, or ammonium or organic amine salt thereof; or a $C_1$—$C_6$ lower alkyl ester thereof.

3. A herbicidally active compound or a resolved optically active isomer thereof having the formula:

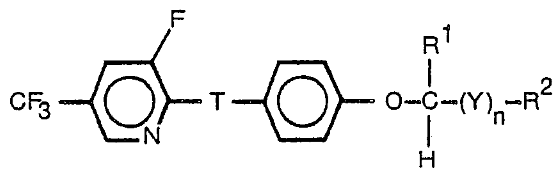

wherein

T is O or S;

Y is an alkyl, alkenyl or alkynyl group containing an even number of from 2 to 18 carbon atoms;

n is 0 or 1;

$R^1$ is a $C_1$—$C_3$ alkyl group; and

$R^2$ is a carboxylic acid group; an alkali, alkaline earth metal salt thereof; or a $C_1$—$C_6$ lower alkyl ester or $C_1$—$C_6$ lower alkyl thiolester thereof; or the group

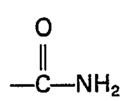 —CN or —$CH_2OH$.

4. A compound as claimed in any one of the preceding Claims wherein n is 0.

5. A compound as claimed in Claim 1 which is 2-(4-(3-fluoro-5-(trifluoromethyl)-2-pyridinyloxy)-phenoxy)propanoic acid or an agriculturally acceptable salt or ester thereof.

6. A compound as claimed in Claim 1 which is 2-(4-(3-fluoro-5-(trifluoromethyl)-2-pyridinyloxy)-phenoxy)propanoic acid, methyl ester.

7. A compound as claimed in Claim 1 which is 2-(4((3-fluoro-5-(trifluoromethyl)-2-pyridinyl)oxy)-phenoxy)propionitrile.

8. The R-enantiomer of a compound as claimed in any one of the preceding Claims.

9. A composition comprising a herbicidally effective amount of a compound as claimed in any one of the preceding Claims together with an inert carrier or diluent.

10. A composition as claimed in Claim 9 which comprises a herbicidally effective amount of 2-(4-(3-fluoro-5-(trifluoromethyl)-2-pyridinyloxy)phenoxy)propanoic acid, methyl ester.

11. A method of controlling undesired plant growth which comprises applying to the locus of the plants a composition as claimed in Claim 9 or Claim 10.

12. A method as claimed in Claim 11 wherein the composition is applied postemergently to grassy weeds.

13. A method as claimed in Claim 11 wherein the composition is applied at a dosage rate sufficient to provide from 0.011 to 22.4 Kgs/hectare (0.01 to 20 pounds per acre) of the active ingredient.

14. A method as claimed in Claim 13 wherein the composition is applied to perennial grassy weeds.

15. A method as claimed in Claim 11 wherein the composition is applied preemergently.

16. A method as claimed in Claim 15 wherein the composition is incorporated into the soil, prior to planting a broadleaf crop, in an effective amount, but less than a phytotoxic amount with respect to the broadleaf crop, to control grassy weeds in the broadleaf crop.

17. A method as claimed in Claim 15 wherein the composition is applied preemergently to control undesired grassy vegetation in the presence of wheat.

18. A process for the preparation of a compound of the formula:

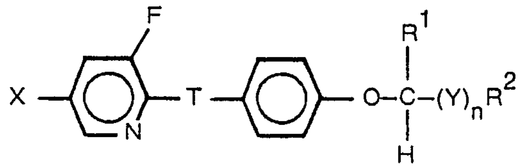

wherein X, T, Y, n, $R^1$ and $R^2$ are as defined in Claim 1, which process comprises reacting a compound of the formula:

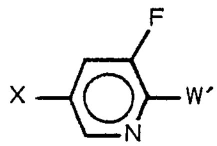

wherein W' is F, Cl or Br and X is as defined in Claim 1 with a compound having the formula:

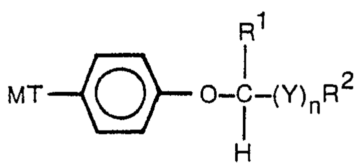

wherein M is hydrogen or a metallic cation, and T, Y, n, $R^1$ and $R^2$ are as defined in Claim 1, in the presence of a base.

19. A process for the preparation of a compound of the formula:

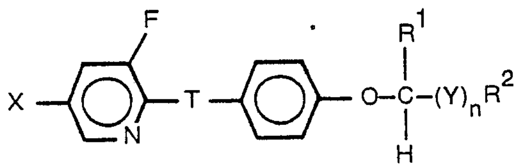

wherein X, T, Y, n, $R^1$ and $R^2$ are as defined in Claim 1, which process comprises reacting a compound having the formula:

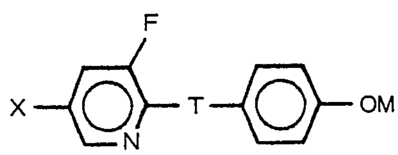

wherein M is H or a metallic cation and X and T are as defined in Claim 1 with a compound having the formula:

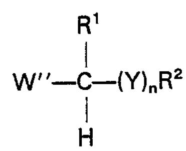

wherein W'' is Cl or R and Y, n, $Br^1$ and $R^2$ are as defined in Claim 1, in the presence of a base.

20. A process for the preparation of a compound of the formula:

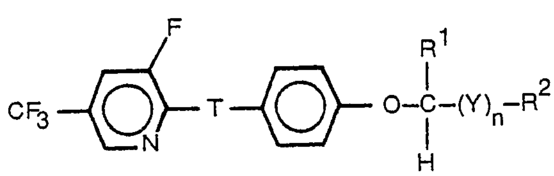

wherein T, Y, n, $R^1$ and $R^2$ are as defined in Claim 3, which process comprises reacting a compound having the formula:

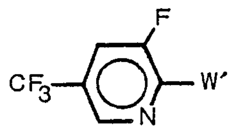

wherein W′ is F, Cl or Br with a compound of the formula:

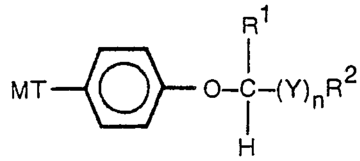

wherein M is hydrogen or a metallic cation, and T, Y, n, $R^1$ and $R^2$ are as defined in Claim 3, in the presence of a base.

21. A process for the preparation of a compound of the formula:

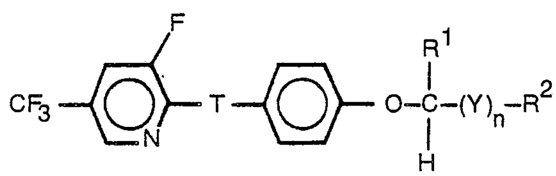

wherein T, Y, n, $R^1$ and $R^2$ are as defined in Claim 3, which process comprises reacting a compound having the formula:

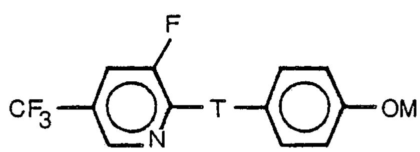

wherein M is H or a metallic cation and T is as defined in Claim 1 with a compound having the formula:

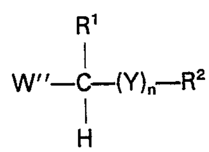

wherein W′′ is Cl or Br and Y, n, $R^1$ and $R^2$ are as defined in Claim 3, in the presence of a base.

22. A compound of the formula

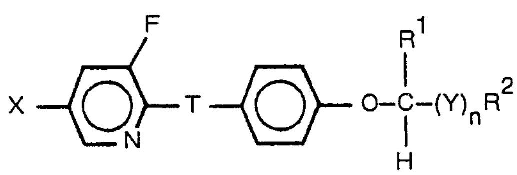

wherein X, T, Y, n, $R^1$ and $R^2$ are as defined in Claim 1, whenever prepared using as an intermediate in the preparation thereof a compound of the formula

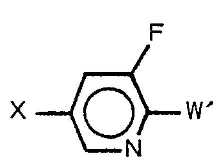

wherein W′ is F, Cl or Br and X is as defined in Claim 1.

23. A compound of the formula

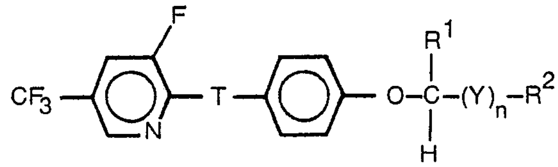

wherein T, Y, n, $R^1$ and $R^2$ are as defined in Claim 3, whenever prepared using as an intermediate in the preparation thereof a compound of the formula:

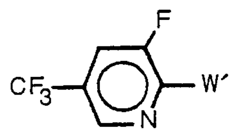

wherein W′ is F, Cl or Br.

24. A compound of the formula:

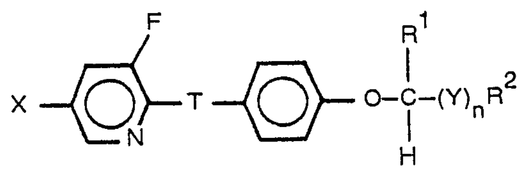

wherein X, T, Y, n, $R^1$ and $R^2$ are as defined in Claim 1, whenever prepared using as an intermediate in the preparation thereof a compound of the formula:

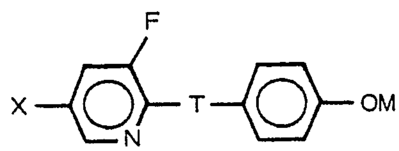

wherein X and T are as defined in Claim 1 and M is H or a metallic cation.

25. A compound of the formula:

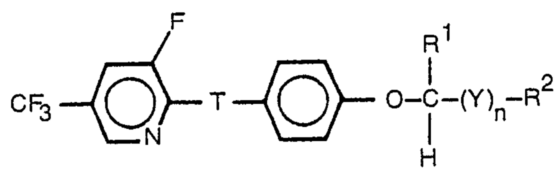

wherein T, Y, n, $R^1$ and $R^2$ are as defined in Claim 3, whenever prepared using as an intermediate in the preparation thereof a compound of the formula:

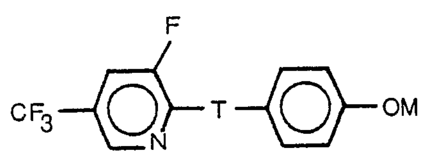

wherein T is as defined in Claim 1 and M is H or a metallic cation.

26. A method of killing or controlling undesired vegetation, which method comprises providing in the vegetation a compound of the formula

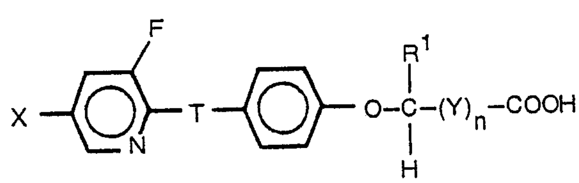

wherein X, T, R′, Y and n are as defined in Claim 1.

27. A method as claimed in Claim 26 which comprises providing in the vegetation a compound of the formula

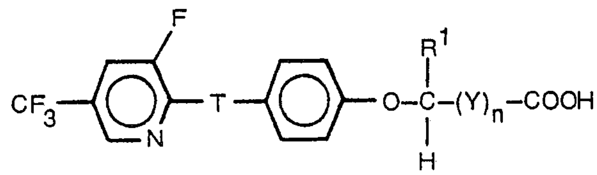

wherein T, $R^1$, Y and n are as defined in claim 1.

28. A method as claimed in Claim 26 which comprises providing in the vegetation 2-(4-(3-fluoro-5-(trifluoromethyl)-2-pyridinyloxy)-phenoxy)propanoic acid.

**Claims for the Contracting State: AT**

1. A process for the preparation of a compound of the formula:

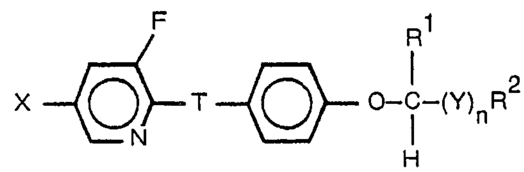

wherein

X is $CF_3$, $CHF_2$ or $CClF_2$;

T is O or S;

Y is an alkyl, alkenyl or alkynyl group containing an even number of from 2 to 18 carbon atoms;

n is 0 or 1;

$R^1$ is a $C_1$—$C_3$ alkyl group; and

$R^2$ is a carboxyl group; an alkali, alkaline earth metal, ammonium or organic amine salt thereof; or an organic group that can be hydrolyzed and/or oxidized in plants or the soil to a carboxyl group that is in undissociated and/or dissociated form which process comprises reacting a compound of the formula:

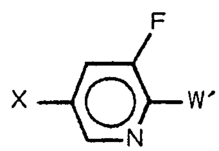

wherein W' is F, Cl or Br and X is as defined above 1 with a compound of the formula:

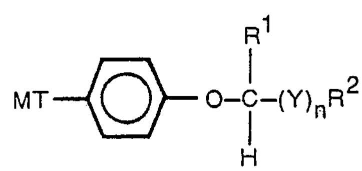

wherein M is hydrogen or a metallic cation, and T, Y, n, $R^1$ and $R^2$ are as defined above, in the presence of a base.

2. A process for the preparation of a compound of the formula:

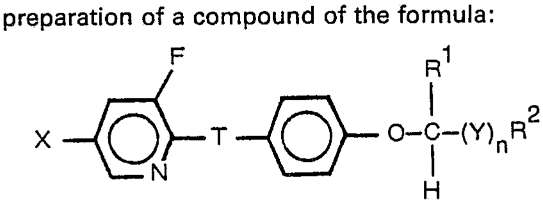

wherein X, T, Y, n, $R^1$ and $R^2$ are as defined in Claim 1, which process comprises reacting a compound having the formula:

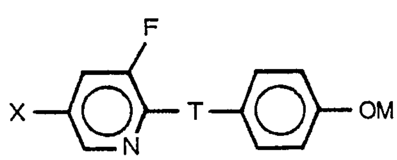

wherein M is H or a metallic cation and X and T are as defined in Claim 1 with a compound having the formula:

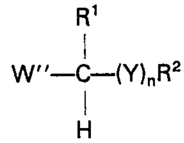

wherein W'' is Cl or Br and Y, n, $R^1$ and $R^2$ are as defined in Claim 1, in the presence of a base.

3. A process for the preparation of a compound of the formula:

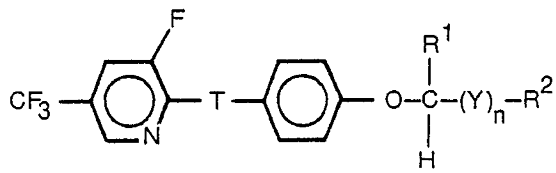

wherein

T is O or S;

Y is alkyl, alkenyl or alkynyl group containing an even number of from 2 to 18 carbon atoms;

n is 0 or 1;

$R^1$ is a $C_1$—$C_3$ alkyl group; and

$R^2$ is a carboxylic acid group; an alkali, alkaline earth metal salt thereof; or a $C_1$—$C_6$ lower alkyl ester or $C_1$—$C_6$ lower alkyl thiolester thereof; or the group

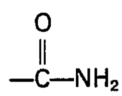 —CN or —$CH_2OH$.

which process comprises reacting a compound of the formula:

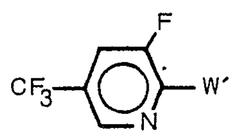

wherein W' is F, Cl or Br with a compound of the formula:

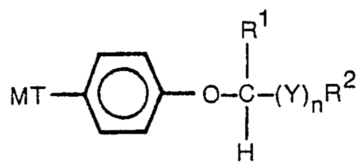

wherein M is hydrogen or a metallic cation, and T, Y, n, $R^1$ and $R^2$ are as defined above, in the presence of a base.

4. A process for the preparation of a compound of the formula:

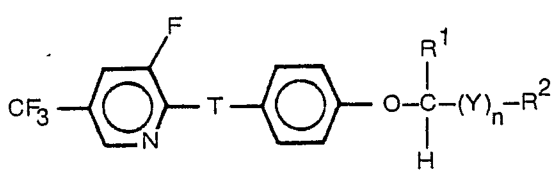

wherein T, Y, n, $R^1$ and $R^2$ are as defined in Claim 3, which process comprises reacting a compound having the formula:

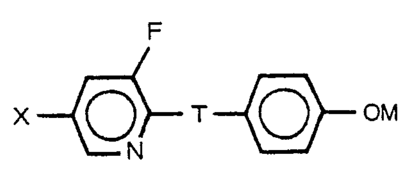

wherein M is H or a metallic cation and T is as defined in Claim 3 with a compound having the formula:

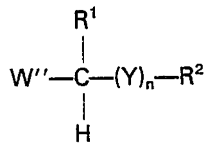

wherein W'' is Cl or Br and R' Y, n and $R^2$ as defined in Claim 3, in the presence of a base.

5. A composition comprising a herbicidally effective amount of a compound or a resolved optically active isomer thereof having the formula:

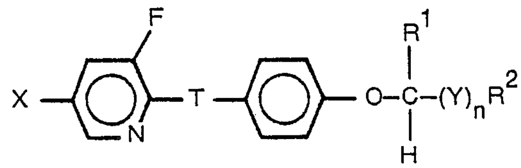

wherein X, T, Y, n, $R^1$ and $R^2$ are as defined in Claim 1 together with an inert diluent or carrier.

6. A composition as claimed in Claim 5 wherein n is 0.

7. A composition as claimed in Claim 5 comprising a herbicidally effective amount of a compound, or a resolved optically active isomer thereof, corresponding to the formula of claim 5, where X is $CF_3$ together with an inert diluent or carrier.

8. A composition as claimed in Claim 7 comprising a herbicidally-effective amount of 2-(4-(3-fluoro-5-(trifluoromethyl)-2-pyridyloxy)phenoxy)propanoic acid, methyl ester.

9. A method for controlling undesired plant growth which comprises applying to the locus of the plants a composition as claimed in any one of Claims 5 to 8.

10. A method as claimed in Claim 9 wherein the composition is applied postemergently to grassy weeds.

11. A method as claimed in Claim 10 wherein the composition is applied at a dosage rate sufficient to provide from 0.011 to 22.4 Kgs/hectare (0.01 to 20 pounds per acre) of the active ingredient.

12. A method as claimed in Claim 11 wherein the composition is applied to perennial grassy weeds.

13. A method as claimed in Claim 9 wherein the compound is applied preemergently.

14. A method as claimed in Claim 13 wherein the compound is incorporated into the soil, prior to planting a broadleaf crop, in an effective amount, but less than a phytotoxic amount with respect to the broadleaf crop, to control grassy weeds in the broadleaf crop.

15. A method as claimed in Claim 14 wherein the compound is applied preemergently to control undesired grassy vegetation in the presence of wheat.

16. A method of killing or controlling undesired vegetation, which method comprises providing in the vegetation a compound of the formula

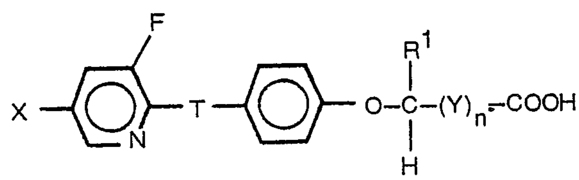

wherein

X, T, R' Y and n are as defined in Claim 1.

17. A method as claimed in claim 16 which comprises providing in the vegetation a compound of the formula

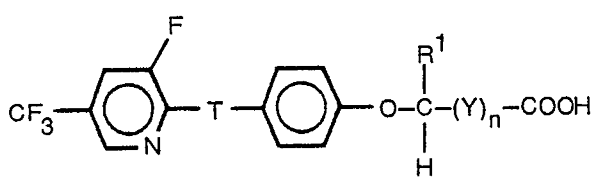

wherein T, R', Y and n are as defined in Claim 1.

18. A method as claimed in Claim 16 which comprises providing in the vegetation 2-(4-(3-fluoro-5-(trifluoromethyl)-2-pyridinyloxy)-phenoxy)propanoic acid.

**Patentansprüche für die Vertragsstaaten: BE CH DE FR IT LI NL SE**

1. Herbizid wirksame Verbindung oder ein aufgetrenntes optisch aktives Isomeres davon mit der Formel:

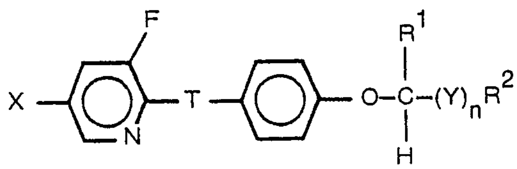

worin

X $CF_3$, $CHF_2$ oder $CClF_2$ ist;

T O oder S ist;

Y eine Alkyl-, Alkenyl- oder Alkynylgruppe mit einer geraden Zahl von 2 bis 18 Kohlenstoffatomen ist;

n 0 oder 1 ist;

$R^1$ eine $C_1$—$C_3$-Alkylgruppe ist; und

$R^2$ eine Carboxylgruppe; ein Alkali-, Erdalkalimetall-, Ammoniumsalz oder ein Salz eines organischen Amins davon ist; oder eine organische Gruppe, die in Pflanzen oder dem Boden zu einer Carboxylgruppe, die in undissoziierter und/oder dissoziierter Form vorliegt, hydrolysiert und/oder oxidiert werden kann.

2. Herbizid wirksame Verbindung oder ein aufgetrenntes, optisch aktives Isomeres davon mit der Formel:

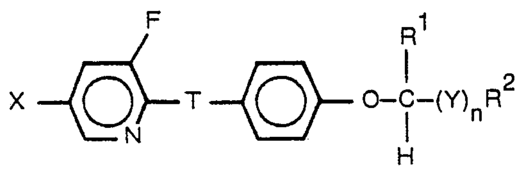

worin

X $CF_3$, $CHF_2$ oder $CClF_2$ ist;

T O oder S ist;

Y eine Alkyl-, Alkenyl- oder Alkynylgruppe mit einer geraden Zahl von 2 bis 18 Kohlenstoffatomen ist;

n 0 oder 1 ist;

$R^1$ eine $C_1$—$C_3$-Alkylgruppe ist; und

$R^2$ eine Carbonsäuregruppe; ein Alkali-, Erdalkalimetall- oder Ammoniumsalz oder ein Salz eines organischen Amins davon ist; oder ein $C_1$—$C_6$-Niederalkylester davon.

3. Herbizid wirksame Verbindung oder ein aufgetrenntes optisch aktives Isomeres davon mit der Formel:

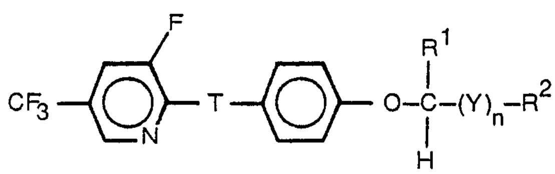

worin

T O oder S ist;

Y eine Alkyl-, Alkenyl- oder Alkynylgruppe mit einer geraden Zahl von 2 bis 18 Kohlenstoffatomen ist;

n 0 oder 1 ist;

$R^1$ eine $C_1$—$C_3$-Alkylgruppe ist; und

$R^2$ eine Carbonsäuregruppe; ein Alkali- oder Erdalkalimetall davon; oder ein $C_1$—$C_6$-Niederalkylester oder $C_1$—$C_6$-Niederalkylthioester davon ist; oder die Gruppe

$$-\overset{O}{\overset{\|}{C}}-NH_2, -CN \text{ oder } -CH_2OH.$$

4. Verbindung nach einem der vorhergehenden Ansprüche, dadurch gekennzeichnet, daß n = 0.

5. Verbindung nach Anspruch 1, dadurch gekennzeichnet, daß sie 2-(4-(3-Fluor-5-(trifluormethyl)-2-pyridinyloxy)-phenoxy)-propionsäure oder ein landwirtschaftlich akzeptierbares Salz oder Ester davon ist.

6. Verbindung nach Anspruch 1, dadurch gekennzeichnet, daß sie 2-(4-(3-fluor-5-(trifluormethyl)-2-pyridinyloxy)-phenoxy)-propionsäuremethylester ist.

7. Verbindung nach Anspruch 1, dadurch gekennzeichnet, daß sie 2-(4-(3-fluor-5-(trifluormethyl)-2-pyridinyl)-oxy)-phenoxy)-propionsäurenitril ist.

8. Das R-Enantiomere einer Verbindung nach einem der vorhergehenden Ansprüche.

9. Zusammensetzung, enthaltend eine herbizid wirksame Menge einer der in einem der vorhergehenden Ansprüche beanspruchten Verbindung, zusammen mit einem inerten Träger oder Verdünnungsmittel.

10. Zusammensetzung nach Anspruch 9, dadurch gekennzeichnet, daß sie eine herbizid wirksame Menge von 2-(4-(3-fluor-5-(trifluormethyl)-2-pyridinyloxy)-phenoxy)-propionsäuremethylester enthält.

11. Verfahren zur Kontrolle unerwünschten Pflanzenwachstums, dadurch gekennzeichnet, daß man an den Ort der Pflanzen eine Zusammensetzung nach Anspruch 9 oder 10 appliziert.

12. Verfahren nach Anspruch 11, dadurch gekennzeichnet, daß die Zusammensetzung an grasigen Unkräutern nach der Entwicklung appliziert wird.

13. Verfahren nach Anspruch 11, dadurch gekennzeichnet, daß die Zusammensetzung in einer Dosis appliziert wird, die ausreicht, um 0,011 bis 22,4 kg/ha (0,01 bis 20 pounds/acre) des aktiven Bestandteils bereitzustellen.

14. Verfahren nach Anspruch 13, dadurch gekennzeichnet, daß die Zusammensetzung an perennierenden grasigen Unkräutern appliziert wird.

15. Verfahren nach Anspruch 11, dadurch gekennzeichnet, daß die Zusammensetzung vor der Entwicklung appliziert wird.

16. Verfahren nach Anspruch 15, dadurch gekennzeichnet, daß die Zusammensetzung vor der Anpflanzung von breitblättrigen Pflanzen in den Boden in einer wirksamen Menge eingebracht wird, aber in einer geringeren Menge als die phytotoxische Menge im Hinblick auf die breitblättrigen Pflanzen, um grasige Unkräuter innerhalb des breitblättrigen Pflanzengutes zu kontrollieren.

17. Verfahren nach Anspruch 15, dadurch gekennzeichnet, daß die Verbindung vor der Entwicklung appliziert wird, um unerwünschte grasige Vegetation in Gegenwart von Weizen zu kontrollieren.

18. Verfahren zur Herstellung einer Verbindung der Formel:

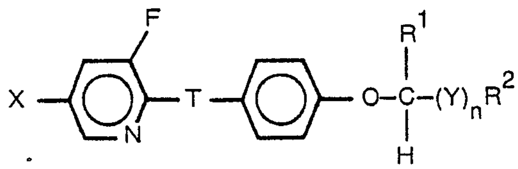

worin X, T, Y, n, $R^1$ und $R^2$ wie in Anspruch 1 definiert sind, dadurch gekennzeichnet, daß man eine Verbindung der Formel:

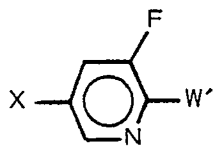

worin W' F, Cl oder Br ist und X wie in Anspruch 1 definiert ist, mit einer Verbindung der Formel:

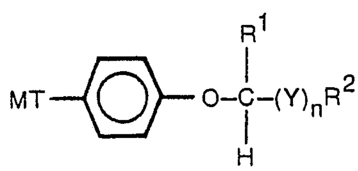

worin M Wasserstoff oder ein metallisches Kation ist, und T, Y, n, $R^1$ und $R^2$ wie in Anspruch 1 definiert sind, in Gegenwart einer Base umsetzt.

19. Verfahren zur Herstellung einer Verbindung der Formel:

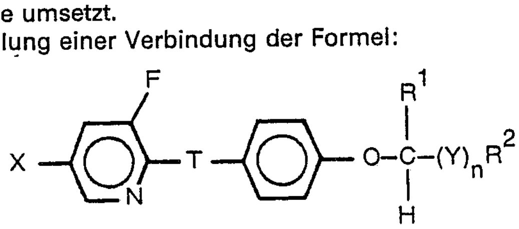

worin X, T, Y, n, $R^1$ und $R^2$ wie in Anspruch 1 definiert sind, dadurch gekennzeichnet, daß man eine Verbindung der Formel:

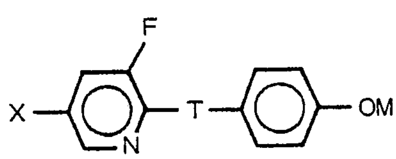

worin M H oder ein metallisches Kation ist, und X und T wie in Anspruch 1 definiert sind, mit einer Verbindung der Formel:

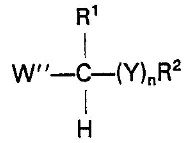

worin W'' Cl oder Br ist und Y, n, $R^1$ und $R^2$ wie in Anspruch 1 definiert sind, in Gegenwart einer Base umsetzt.

20. Verfahren zur Herstellung einer Verbindung der Formel:

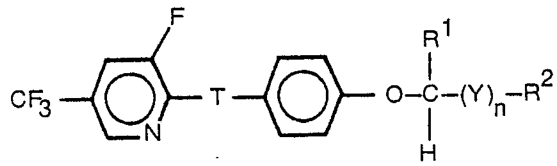

worin T, Y, n, $R^1$ und $R^2$ wie in Anspruch 3 definiert sind, dadurch gekennzeichnet, daß man eine Verbindung der Formel:

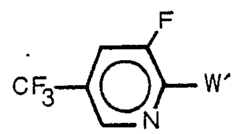

worin W' F, Cl oder Br ist, mit einer Verbindung der Formel:

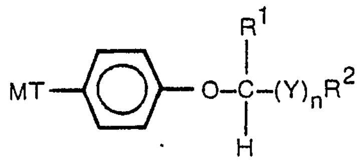

worin M Wasserstoff oder ein metallisches Kation ist, und T, Y, n, $R^1$ und $R^2$ wie in Anspruch 3 definiert sind, in Gegenwart einer Base umsetzt.

21. Verfahren zur Herstellung einer Verbindung der Formel:

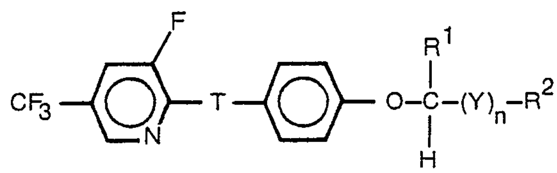

worin T, Y, n, $R^1$ und $R^2$ wie in Anspruch 3 definiert sind, dadurch gekennzeichnet, daß man eine Verbindung der Formel:

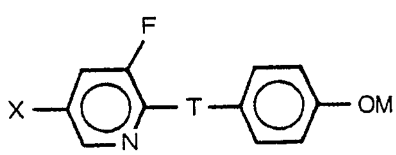

worin M H oder ein metallisches Kation ist, und T wie in Anspruch 1 definiert ist, mit einer Verbindung der Formel:

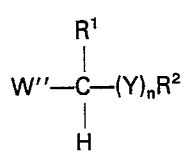

worin W'' Cl oder Br ist und Y, n, $R^1$ und $R^2$ wie in Anspruch 3 definiert sind, in Gegenwart einer Base umsetzt.

22. Verbindung der Formel:

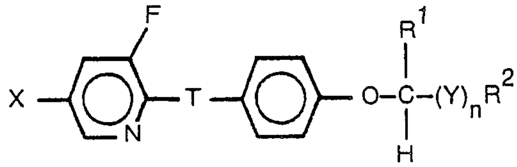

worin X, T, Y, n, $R^1$ und $R^2$ wie in Anspruch 1 definiert sind, hergestellt unter Verwendung eines Zwischenproduktes der Formel

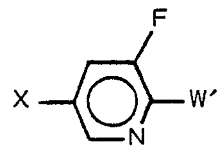

worin W' F, Cl oder Br ist und X wie in Anspruch 1 definiert ist.

23. Verbindung der Formel:

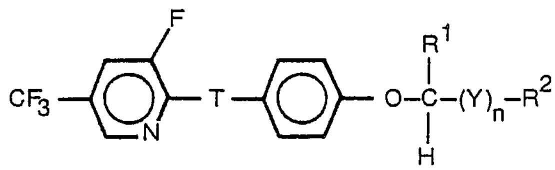

worin R, Y, n, $R^1$ und $R^2$ wie in Anspruch 3 definiert sind, hergestellt unter Verwendung eines Zwischenproduktes der Formel:

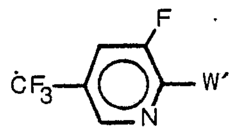

worin W' F, Cl oder Br ist.

24. Verbindung der Formel:

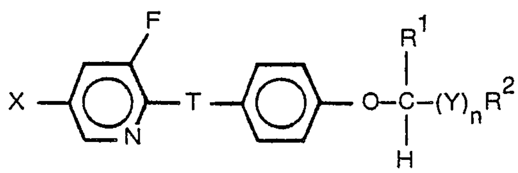

worin X, T, Y, n, $R^1$ und $R^2$ wie in Anspruch 1 definiert sind, hergestellt unter Verwendung eines Zwischenproduktes der Formel:

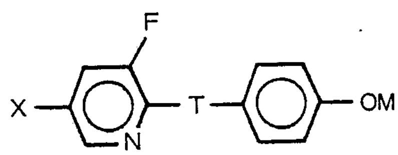

worin X und T wie in Anspruch 1 definiert sind, und M H oder ein metallisches Kation ist.

25. Verbindung der Formel:

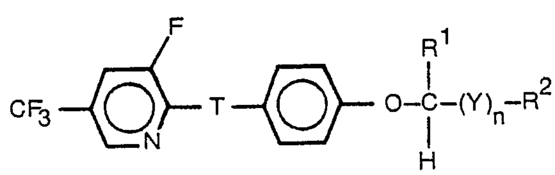

worin T, Y, n, $R^1$ und $R^2$ wie in Anspruch 3 definiert sind, hergestellt unter Verwendung eines Zwischenproduktes der Formel:

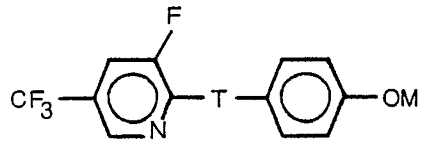

worin T wie in Anspruch 1 definiert ist, und M H oder ein metallisches Kation ist.

26. Verfahren zur Abtötung oder Kontrolle unerwünschter Vegetation, dadurch gekennzeichnet, daß man in der Vegetation eine Verbindung der Formel vorsieht

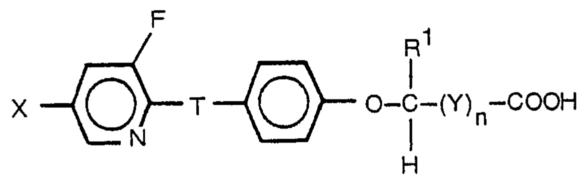

worin X, T, $R^1$, Y und n wie in Anspruch 1 definiert sind.

27. Verfahren nach Anspruch 26, dadurch gekennzeichnet, daß man in der Vegetation eine Verbindung der Formel vorsieht:

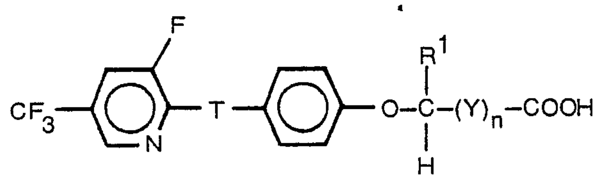

worin T, $R^1$, Y und n wie in Anspruch 1 definiert sind.

28. Verfahren nach Anspruch 26, dadurch gekennzeichnet, daß man in der Vegetation 2-(4-(3-Fluor-5-(trifluormethyl)-2-pyridinyloxy)-phenoxy)-propionsäure vorsieht.

**Patentansprüche für den Vertragsstaat: AT**

1. Verfahren zur Herstellung einer Verbindung der Formel:

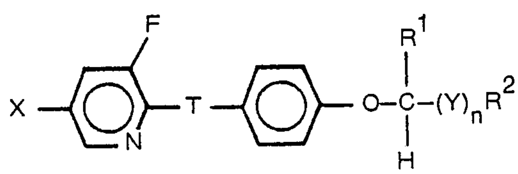

worin

X $CF_3$, $CHF_2$ oder $CClF_2$ ist;

T O oder S ist;

Y eine Alkyl-, Alkenyl- oder Alkynylgruppe mit einer geraden Zahl von 2 bis 18 Kohlenstoffatomen ist;

n = 0 oder 1;

$R^1$ eine $C_1$—$C_3$-Alkylgruppe ist; und

$R^2$ eine Carboxylgruppe ist; ein Alkali-, Erdalkalimetall-, Ammoniumsalz oder ein Salz eines organischen Amins davon ist; oder eine organische Gruppe, die in Pflanzen oder im Boden zu einer Carboxylgruppe, die in undissoziierter und/oder dissoziierter Form vorliegt, hydrolysiert und/oder oxidiert werden kann, dadurch gekennzeichnet, daß man eine Verbindung der Formel:

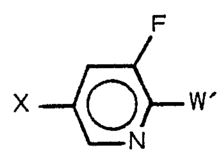

worin W′ F, Cl oder Br ist, und X wie oben definiert ist, mit einer Verbindung der Formel:

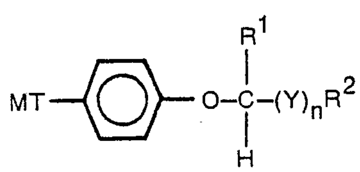

worin M Wasserstoff oder ein metallisches Kation ist, und T, Y, n, $R^1$ und $R^2$ wie oben definiert sind, in Gegenwart einer Base umsetzt.

2. Verfahren zur Herstellung einer Verbindung der Formel:

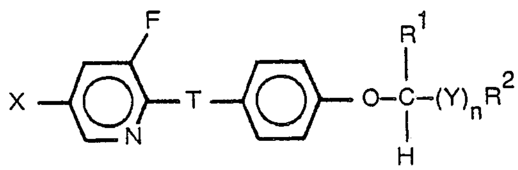

worin X, T, Y, n, $R^1$ und $R^2$ wie in Anspruch 1 definiert sind, dadurch gekennzeichnet, daß man eine Verbindung der Formel:

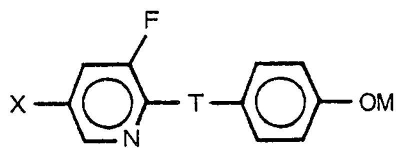

worin M H oder ein metallisches Kation ist, und T und X wie in Anspruch 1 definiert ist, mit einer Verbindung der Formel:

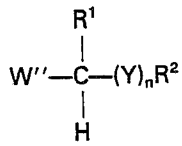

worin W'' Cl oder Br ist und Y, n, $R^1$ und $R^2$ wie in Anspruch 1 definiert sind, in Gegenwart einer Base umsetzt.

3. Verfahren zur Herstellung einer Verbindung der Formel:

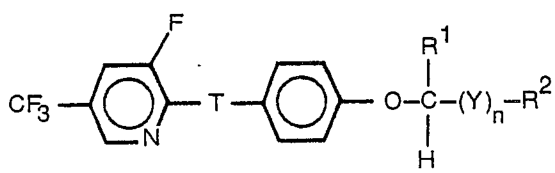

worin

T O oder S ist;

Y eine Alkyl-, Alkenyl- oder Alkynylgruppe mit einer geraden Zahl von 2 bis 18 Kohlenstoffatomen ist;

n = 0 oder 1;

$R^1$ eine $C_1$—$C_3$-Alkylgruppe ist; und

$R^2$ eine Carbonsäuregruppe ist; ein Alkali- oder Erdalkalimetallsalz davon; oder ein $C_1$—$C_6$-Niederalkylester oder $C_1$—$C_6$-Niederalkylthioester davon ist; oder die Gruppe

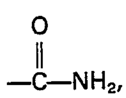 , —CN oder —$CH_2OH$,

dadurch gekennzeichnet, daß man eine Verbindung der Formel:

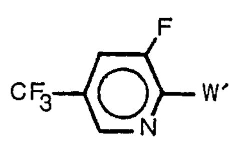

worin W' F, Cl oder Br ist, mit einer Verbindung der Formel:

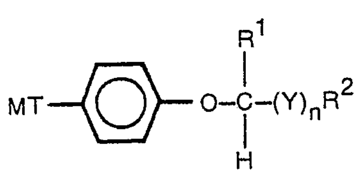

worin M Wasserstoff oder ein metallisches Kation ist, und T, Y, n, $R^1$ und $R^2$ wie oben definiert sind, in Gegenwart einer Base umsetzt.

4. Verfahren zur Herstellung einer Verbindung der Formel:

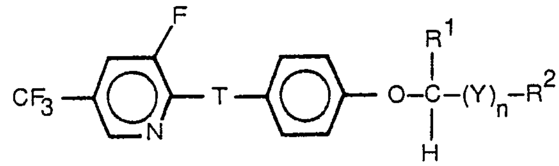

worin T, Y, n, $R^1$ und $R^2$ wie in Anspruch 3 definiert sind, dadurch gekennzeichnet, daß man eine Verbindung der Formel:

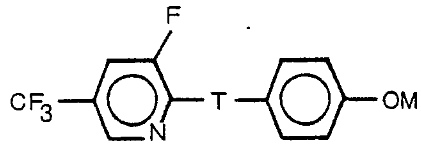

worin M H oder ein metallisches Kation ist, und T wie in Anspruch 3 definiert ist, mit einer Verbindung der Formel:

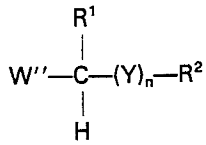

worin W'' Cl oder Br ist, und $R^1$, Y, n und $R^2$ wie in Anspruch 3 definiert sind, in Gegenwart einer Base umsetzt.

5. Zusammensetzung, enthaltend eine herbizid wirksame Menge einer Verbindung oder ein aufgetrenntes, optisch aktives Isomeres davon mit der Formel:

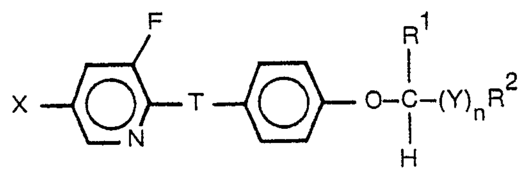

worin X, T, Y, n, $R^1$ und $R^2$ wie in Anspruch 1 definiert sind, zusammen mit einem inerten Verdünnungsmittel oder Träger.

6. Zusammensetzung nach Anspruch 5, dadurch gekennzeichnet, daß $n = 0$.

7. Zusammensetzung nach Anspruch 5, dadurch gekennzeichnet, daß sie eine herbizid wirksame Menge einer Verbindung oder eines aufgetrennten optisch aktiven Isomeres davon, entsprechend der Formel nach Anspruch 5, worin X $CF_3$ ist, enthält, zusammen mit einem inerten Verdünnungsmittel oder Träger.

8. Zusammensetzung nach Anspruch 7, dadurch gekennzeichnet, daß sie eine herbizid wirksame Menge von 2-(4-(3-Fluor-5-(trifluormethyl)-2-pyridyloxy)-phenoxy)-propionsäuremethylester enthält.

9. Verfahren zur Kontrolle unerwünschten Pflanzenwachstums, dadurch gekennzeichnet, daß man an den Ort der Pflanzen eine Zusammensetzung nach einem der Ansprüche 5 bis 8 appliziert.

10. Verfahren nach Anspruch 9, dadurch gekennzeichnet, daß die Zusammensetzung an grasigen Unkräutern nach der Entwicklung appliziert wird.

11. Verfahren nach Anspruch 10, dadurch gekennzeichnet, daß die Zusammensetzung in einer Dosierung appliziert wird, die ausreicht, um 0,011 bis 22,4 kg/ha (0,01 bis 20 pounds/acre) des aktiven Bestandteiles bereitzustellen.

12. Verfahren nach Anspruch 11, dadurch gekennzeichnet, daß die Zusammensetzung an perennierenden grasigen Unkräutern appliziert wird.

13. Verfahren nach Anspruch 9, dadurch gekennzeichnet, daß die Verbindung vor der Entwicklung appliziert wird.

14. Verfahren nach Anspruch 13, dadurch gekennzeichnet, daß die Verbindung vor der Anpflanzung eines breitblättrigen Pflanzengutes in einer wirksamen Menge in den Boden eingebracht wird, aber in einer Menge, die geringer ist als die phytotoxische Menge im Hinblick auf die breitblättrigen Pflanzen, um grasiges Unkraut in dem breitblättrigen Pflanzengut zu kontrollieren.

15. Verfahren nach Anspruch 14, dadurch gekennzeichnet, daß die Verbindung vor der Entwicklung appliziert wird, um unerwünschte grasige Vegetation in Gegenwart von Weizen zu kontrollieren.

16. Verfahren zur Abtötung oder Kontrolle unerwünschter Vegetation, dadurch gekennzeichnet, daß man in der Vegetation eine Verbindung der Formel vorsieht

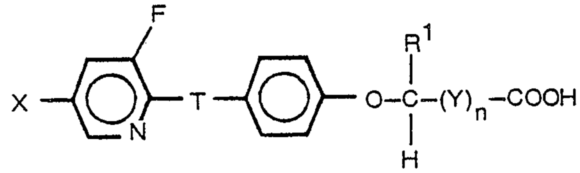

worin X, T, $R^1$, Y und n wie in Anspruch 1 definiert sind.

17. Verfahren nach Anspruch 16, dadurch gekennzeichnet, daß man in der Vegetation eine Verbindung der Formel vorsieht:

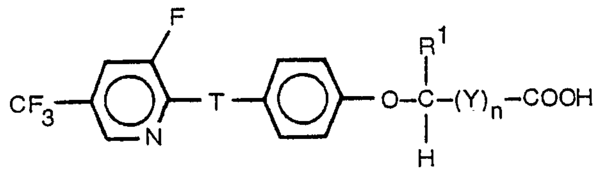

worin T, $R^1$, Y und n wie in Anspruch 1 definiert sind.

18. Verfahren nach Anspruch 16, dadurch gekennzeichnet, daß man in der Vegetation 2-(4-(3-Fluor-5-(trifluormethyl)-2-pyridinyloxy)-phenoxy)-propionsäure vorsieht.

**Revendications pour les Etats contractants: BE CH DE FR IT LI NL SE**

1. Composé actif du point de vue herbicide ou un de ses isomères dédoublés, optiquement actif, répondant à la formule:

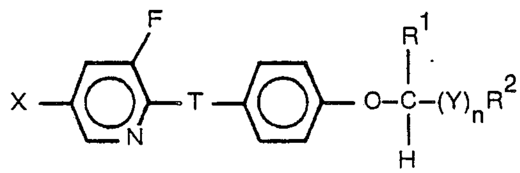

dans laquelle

X est $CF_3$, $CHF_2$ ou $CClF_2$;

T est O ou S;

Y est un groupe alkyle, alcényle ou alcynyle contenant un nombre pair, allant de 2 à 18, d'atomes de carbone;

n vaut 0 ou 1;

$R^1$ est un groupe alkyle en $C_1$—$C_3$; et

$R^2$ est un groupe carboxyle; un de ses sels de métal alcalin, de métal alcalino terreux, d'ammonium ou d'amine organique; ou un groupe organique qui peut être hydrolysé et/ou oxydé dans les plantes ou dans la terre en un groupe carboxyle qui est sous une forme non dissociée et/ou dissociée.

2. Composé actif du point de vue herbicide ou un de ses isomères dédoublés, optiquement actif, répondant à la formule:

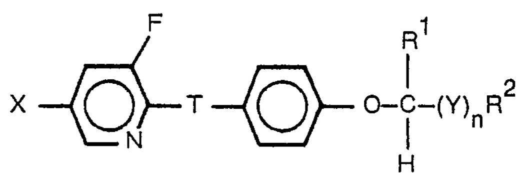

dans laquelle

X est $CF_3$, $CHF_2$ ou $CClF_2$;

T est O ou S;

Y est un groupe alkyle, alcényle ou alcynyle contenant un nombre pair, allant de 2 à 18, d'atomes de carbone;

n vaut 0 ou 1;

$R^1$ est un groupe alkyle en $C_1$—$C_3$; et

$R^2$ est un groupe acide carboxylique; un de ses sels de métal alcalin, de métal alcalino terreux, ou d'ammonium ou d'amine organique; ou un des ses esters d'alkyle inférieur en $C_1$—$C_6$.

3. Composé actif du point de vue herbicide ou un de ses isomères dédoublés, optiquement actif, répondant à la formule:

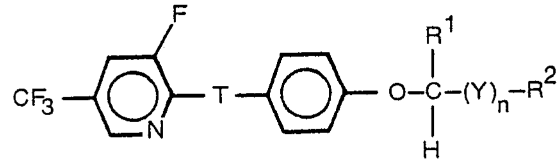

dans laquelle

T est O ou S;

Y est un groupe alkyle, alcényle ou alcynyle contenant un nombre pair, allant de 2 à 18, d'atomes de carbone;

n vaut 0 ou 1;

$R^1$ est un groupe alkyle en $C_1—C_3$; et

$R^2$ est un groupe acide carboxylique; un de ses sels de métal alcalin ou de métal alcalino-terreux; ou un de ses esters d'alkyle inférieur en $C_1—C_6$ ou un de ses thiolesters d'alkyle inférieur en $C_1—C_6$; ou le groupe

$$—\overset{\overset{O}{\|}}{C}—NH_2;\ \text{ou}\ —CN\ \text{ou}\ —CH_2OH.$$

4. Composé selon l'une quelconque des revendications précédentes dans lequel n vaut 0.

5. Composé selon la revendication 1, qui est l'acide 2-(4-(3-fluoro-5-(trifluorométhyl)-2-pyridinyloxy)-phénoxy)propanoique ou un de ses esters ou sels acceptable en agriculture.

6. Composé selon la revendication 1, qui est l'ester méthylique de l'acide 2-(4-(3-fluoro-5-(trifluorométhyl)-2-pyridinyloxy)-phénoxy)propanoique.

7. Composé selon la revendication 1, qui est le 2-(4-((3-fluoro-5-(trifluorométhyl)-2-pyridinyl)oxy)phénoxy)propionitrile.

8. Enantiomère-R d'un composé selon l'une quelconque des revendications précédentes.

9. Composition comprenant une quantité efficace du point de vue herbicide d'un composé selon l'une quelconque des revendications précédentes, conjointement avec un diluant ou un véhicule inerte.

10. Composition selon la revendication 9, qui comprend une quantité efficace du point de vue herbicide de l'ester méthylique de l'acide 2-(4-(3-fluoro-5-(trifluorométhyl)-2-pyridinyloxy)-phénoxy)propanoique.

11. Procédé pour combattre la croissance non désirée des plantes, qui comprend l'application, au lieu où poussent les plantes, d'une composition selon la revendication 9 ou 10.

12. Procédé selon la revendication 11, dans lequel on applique la composition aux mauvaises herbes herbacées en post-levée.

13. Procédé selon la revendication 11, dans lequel on applique la composition à une dose suffisante pour fournir de 0,011 à 22,4 kg/hectare (0,01 à 20 livres par acre) de l'ingrédient actif.

14. Procédé selon la revendication 13, dans lequel on applique la composition à des mauvaises herbes herbacées vivaces.

15. Procédé selon la revendication 11, dans lequel on applique la composition en pré-levée.

16. Procédé selon la revendication 15, dans lequel on incorpore la composition dans la terre, avant la plantation d'une culture à larges feuilles, en une quantité efficace, mais inférieure à une quantité phytotoxique vis-à-vis de la culture à larges feuilles, pour combattre les mauvaises herbes herbacées dans la culture à larges feuilles.

17. Procédé selon la revendication 15, dans lequel on applique le composé en pré-levée, afin de combattre la végétation herbacée non désirée en présence de blé.

18. Procédé pour la préparation d'un composé de formule:

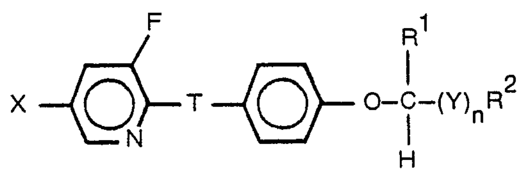

dans laquelle X, T, Y, n, $R^1$ et $R^2$ sont tels que définis dans la revendication 1, qui comprend la réaction d'un composé de formule:

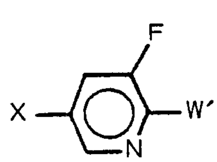

dans laquelle W′ est F, Cl ou Br et X est tel que défini dans la revendication 1, avec un composé répondant à la formule:

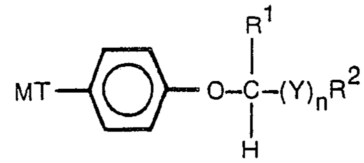

dans laquelle M est l'atome d'hydrogène ou un cation métallique, et T, Y, n, $R^1$ et $R^2$ sont tels que définis dans la revendication 1, en présence d'une base.

19. Procédé pour la préparation d'un composé de formule:

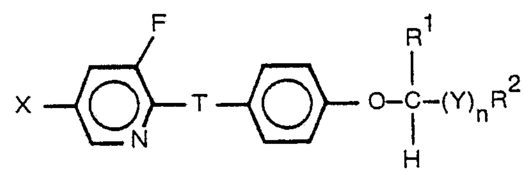

dans laquelle X, T, Y, n, $R^1$ et $R^2$ sont tels que définis dans la revendication 1, qui comprend la réaction d'un composé répondant à la formule:

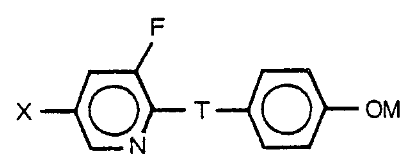

dans laquelle M est H ou un cation métallique et X et T sont tels que définis dans la revendication 1, avec un composé répondant à la formule:

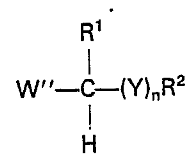

dans laquelle W′′ est Cl ou Br et Y, n, $R^1$ et $R^2$ sont tels que définis dans la revendication 1, en présence d'une base.

20. Procédé pour la préparation d'un composé de formule:

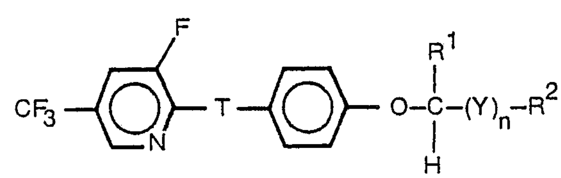

dans laquelle T, Y, n, $R^1$ et $R^2$ sont tels que définis dans la revendication 3, qui comprend la réaction d'un composé répondant à la formule:

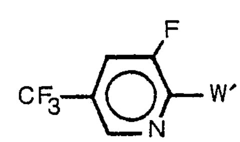

dans laquelle W′ est F, Cl ou Br, avec un composé de formule:

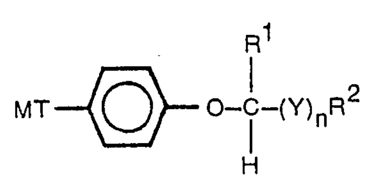

dans laquelle M est un hydrogène ou un cation métallique, et T, Y, n, $R^1$ et $R^2$ sont tels que définis dans la revendication 3, en présence d'une base.

21. Procédé pour la préparation d'un composé de formule:

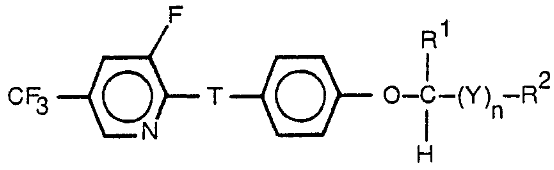

dans laquelle T, Y, n, $R^1$ et $R^2$ sont tels que définis dans la revendication 3, qui comprend la réaction d'un composé répondant à la formule:

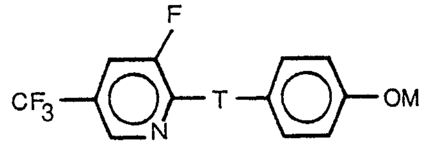

dans laquelle M est H ou un cation métallique et T est tel que défini dans la revendication 1, avec un composé répondant à la formule:

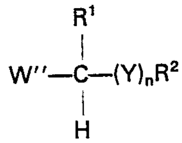

dans laquelle W'' est Cl ou Br et Y, n, $R^1$ et $R^2$ sont tels que définis dans la revendication 3, en présence d'une base.

22. Composé de formule:

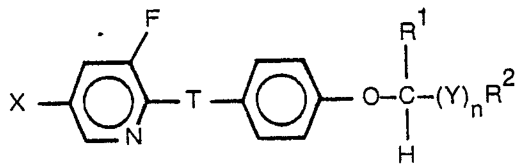

dans laquelle X, T, Y, n, $R^1$ et $R^2$ sont tels que définis dans la revendication 1, préparé en utilisant, à titre d'intermédiaire, un composé de formule:

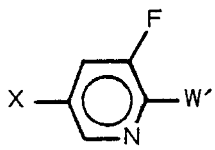

dans laquelle W' est F, Cl ou Br et X est tel que défini dans la revendication 1.

23. Composé de formule:

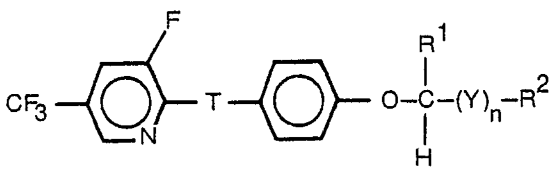

dans laquelle T, Y, n, $R^1$ et $R^2$ sont tels que définis dans la revendication 3, préparé en utilisant comme intermédiaire, un composé de formule:

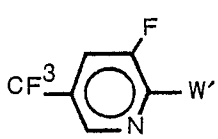

dans laquelle W' est F, Cl ou Br.

24. Composé de formule:

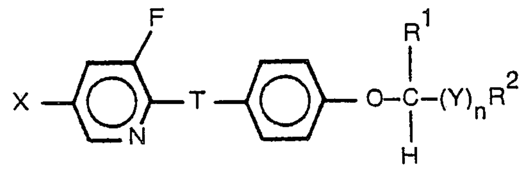

dans laquelle X, T, Y, n, $R^1$ et $R^2$ sont tels que définis dans la revendication 1, préparé, en utilisant à titre d'intermédiaire, un composé de formule:

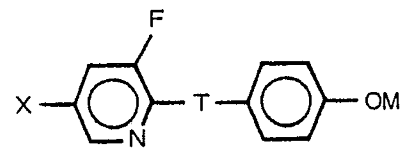

dans laquelle X et T sont tels que définis dans la revendication 1, et M est H ou un cation métallique.

25. Composé de formule:

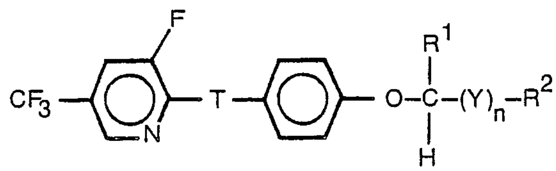

dans laquelle T, Y, n, $R^1$ et $R^2$ sont tels que définis dans la revendication 3, préparé en utilisant, à titre d'intermédiaire, un composé de formule:

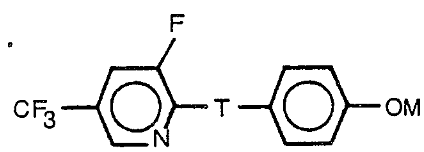

dans laquelle T est tel que défini dans la revendication 1, et M est H ou un cation métallique.

26. Procédé pour détruire ou combattre la végétation non désirée, qui comprend l'apport à la végétation, d'un composé de formule:

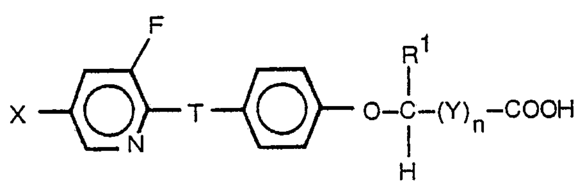

dans laquelle X, T, $R^1$, Y et n sont tels que définis dans la revendication 1.

27. Procédé selon la revendication 26, qui comprend l'apport à la végétation, d'un composé de formule:

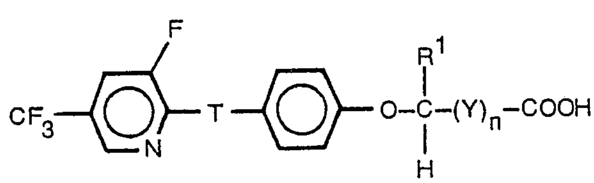

dans laquelle T, $R^1$, Y et n sont tels que définis dans la revendication 1.

28. Procédé selon la revendication 26, qui comprend l'apport à la végétation de l'acide 2-(4-(3-fluoro-5-(trifluorométhyl)-2-pyridinyloxy)-phénoxy)propanoique.

**Revendications pour l'Etat contractant: AT**

1. Procédé pour la préparation d'un composé de formule:

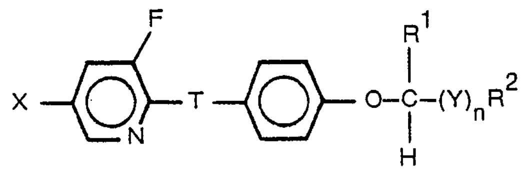

dans laquelle
X est $CF_3$, $CHF_2$ ou $CClF_2$;
T est O ou S;
Y est un groupe alkyle, alcényle ou alcynyle contenant un nombre pair, allant de 2 à 18, d'atomes de carbone;
n vaut 0 ou 1;
$R^1$ est un groupe alkyle en $C_1$—$C_3$; et
$R^2$ est un groupe carboxyle; un de ses sels de métal alcalin, de métal alcalino-terreux, d'ammonium ou d'amine organique; ou un groupe organique qui peut être hydrolysé et/ou oxydé dans les plantes ou dans la terre en un groupe carboxyle qui est sous une forme non dissociée et/ou dissociée, qui comprend la réaction d'un composé de formule:

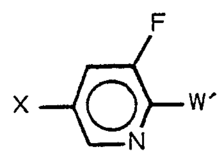

dans laquelle W' est F, Cl ou Br et X est tel que défini ci-dessus, avec un composé de formule:

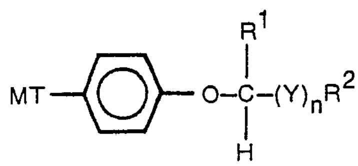

dans laquelle M est un atome d'hydrogène ou un cation métallique et T, Y, n, $R^1$ et $R^2$ sont tels que définis ci-dessus, en présence d'une base.

2. Procédé pour la préparation d'un composé de formule:

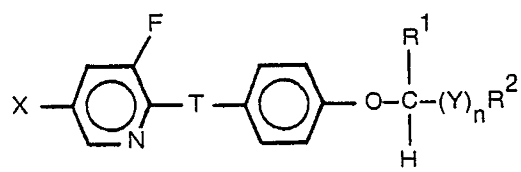

dans laquelle X, T, Y, n, $R^1$ et $R^2$ sont tels que définis dans la revendication 1, qui comprend la réaction d'un composé répondant à la formule:

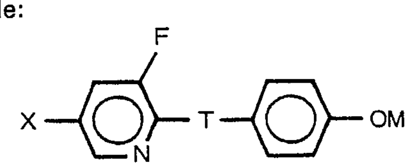

dans laquelle M est H ou un cation métallique, et T et X sont tels que définis dans la revendication 1, avec un composé répondant à la formule:

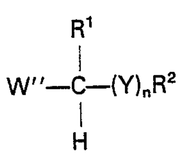

dans laquelle W'' est Cl ou Br et Y, n, $R^1$ et $R^2$ sont tels que définis dans la revendication 1, en présence d'une base.

3. Procédé pour la préparation d'un composé de formule:

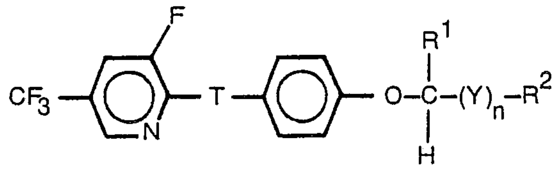

dans laquelle
T est O ou S;
Y est un groupe alkyle, alcényle ou alcynyle contenant un nombre pair, allant de 2 à 18, d'atomes de carbone;
n vaut 0 ou 1;
$R^1$ est un groupe alkyle en $C_1$—$C_3$; et
$R^2$ est un groupe acide carboxylique; un de ses sels de métal alcalin ou de métal alcalino-terreux; ou un de ses esters ou thiolesters d'alkyle inférieur en $C_1$—$C_6$; ou le groupe

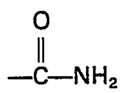 ou —CN ou —$CH_2OH$,

qui comprend la réaction d'un composé répondant à la formule:

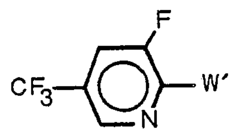

dans laquelle W' est F, Cl ou Br, avec un composé de formule:

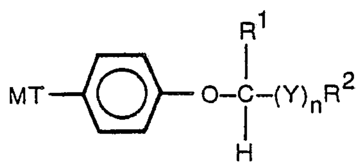

dans laquelle M est un atome d'hydrogène ou un cation métallique, et T, Y, n, $R^1$ et $R^2$ sont tels que définis ci-dessus, en présence d'une base.

4. Procédé pour la préparation d'un composé de formule:

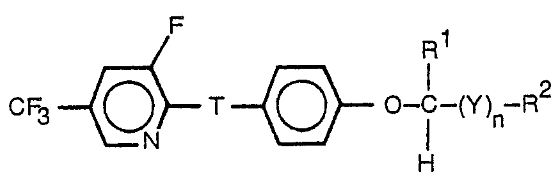

dans laquelle T, Y, n, $R^1$ et $R^2$ sont tels que définis dans la revendication 3, qui comprend la réaction d'un composé ayant la formule:

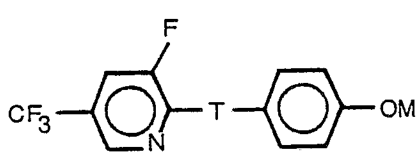

dans laquelle M est H ou un cation métallique et T est tel que défini dans la revendication 3, avec un composé ayant la formule:

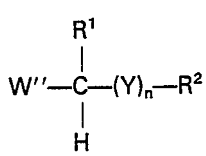

dans laquelle W'' est Cl ou Br et Y, n, $R^1$ et $R^2$ sont tels que définis dans la revendication 3, en présence d'une base.

5. Composition comprenant une quantité efficace du point de vue herbicide d'un composé ou d'un de ses isomères déboublés, optiquement actif, répondant à la formule:

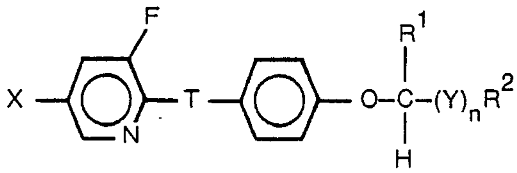

dans laquelle X, T, Y, n, $R^1$ et $R^2$ sont tels que définis dans la revendication 1, conjointement avec un véhicule ou un diluant inerte.

6. Composition selon la revendication 5, dans laquelle n vaut 0.

7. Composition selon la revendication 5, qui comprend une quantité efficace du point de vue herbicide d'un composé ou d'un de ses isomères dédoublés, optiquement actif, répondant à la formule de la revendication 5 dans laquelle X est $CF_3$, conjointement avec un diluant ou un véhicule inerte.

8. Composition selon la revendication 7, qui comprend une quantité efficace du point de vue herbicide de l'ester méthylique de l'acide 2-(4-(3-fluoro-5-(trifluorométhyl)-2-pyridinyloxy)-phénoxy)propanoique.

9. Procédé pour combattre la croissance des plantes non désirées, qui comprend l'application au lieu où poussent les plantes d'une composition selon l'une quelconque des revendications 5 à 8.

10. Procédé selon la revendication 9, dans lequel on applique la composition à des mauvaises herbes herbacées en post-levée.

11. Procédé selon la revendication 10, dans lequel on applique la composition à une dose suffisante pour fournir de 0,011 à 22,4 Kg/hectare (0,01 à 20 livres par acre) de l'ingrédient actif.

12. Procédé selon la revendication 11, dans lequel on applique la composition à des mauvaises herbes herbacées vivaces.

13. Procédé selon la revendication 9, dans lequel on applique le composé en pré-levée.

14. Procédé selon la revendication 13, dans lequel on incorpore le composé dans la terre, avant la plantation d'une culture à larges feuilles, en une quantité efficace, mais inférieure à une quantité phytotoxique par rapport à la culture à larges feuilles, afin de combattre les mauvaises herbes herbacées dans la culture à larges feuilles.

15. Procédé selon la revendication 14, dans lequel on applique le composé en pré-levée, afin de combattre la végétation herbacée non désirée en présence de blé.

16. Procédé pour détruire ou combattre la végétation non désirée, qui comprend l'apport à la végétation, d'un composé de formule:

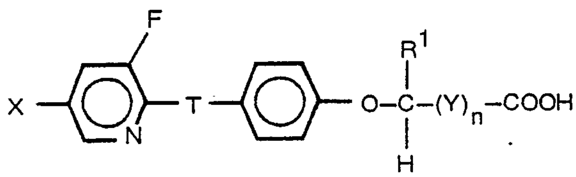

dans laquelle X, T, $R^1$, Y et n sont tels que définis dans la revendication 1.

17. Procédé selon la revendication 16, qui comprend l'apport à la végétation d'un composé de formule:

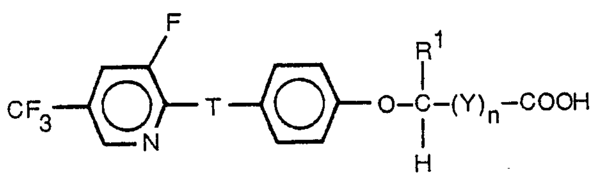

dans laquelle T, $R^1$, Y et n sont tels que définis dans la revendication 1.

18. Procédé selon la revendication 16, qui comprend l'apport à la végétation, de l'acide 2-(4-(3-fluoro-5-(trifluorométhyl)-2-pyridinyloxy)-phénoxy)propanoique.